(19) Europäisches Patentamt
European Patent Office
Office européen des brevets

(11) **EP 4 190 790 A1**

(12) **EUROPEAN PATENT APPLICATION**
published in accordance with Art. 153(4) EPC

(43) Date of publication:
**07.06.2023 Bulletin 2023/23**

(21) Application number: **21849829.3**

(22) Date of filing: **30.07.2021**

(51) International Patent Classification (IPC):
***C07D 498/06*** *(2006.01)* ***C07D 267/02*** *(2006.01)*
***A61K 31/55*** *(2006.01)* ***A61P 35/00*** *(2006.01)*

(52) Cooperative Patent Classification (CPC):
**A61K 31/55; A61P 35/00; C07D 267/02; C07D 498/06**

(86) International application number:
**PCT/CN2021/109452**

(87) International publication number:
**WO 2022/022664 (03.02.2022 Gazette 2022/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.07.2020 CN 202010762484**
**04.08.2020 CN 202010772764**

(71) Applicant: **Chia Tai Tianqing Pharmaceutical Group Co., Ltd.**
**Lianyungang City, Jiangsu 222062 (CN)**

(72) Inventors:
- **HU, Yanbin**
  **Shanghai 200131 (CN)**
- **LI, Gang**
  **Shanghai 200131 (CN)**
- **HU, Lihong**
  **Shanghai 200131 (CN)**
- **DING, Charles Z.**
  **Shanghai 200131 (CN)**
- **CHEN, Shuhui**
  **Shanghai 200131 (CN)**

(74) Representative: **Müller Schupfner & Partner Patent- und Rechtsanwaltspartnerschaft mbB**
**Bavariaring 11**
**80336 München (DE)**

# (54) INDOLO HEPTAMYL OXIME ANALOG CRYSTAL AS PARP INHIBITOR AND METHOD FOR PREPARING SAME

(57) Disclosed is a type of indolo heptamyl oxime analog crystalline form as a PARP inhibitor and a method for preparing the same, specifically relating to a crystalline form of a compound of formula (I) and a method for preparing the same.

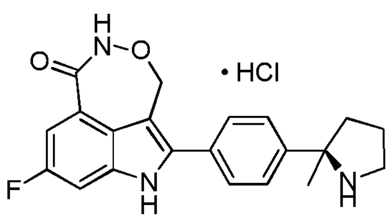

(I)

EP 4 190 790 A1

## Description

## CROSS-REFERENCE TO RELATED APPLICATIONS

The present application claims the benefits and priority of the Chinese Patent Application No. 202010762484.9 filed with National Intellectual Property Administration, PRC on July 31, 2020 and the Chinese Patent Application No. 202010772764.8 filed with National Intellectual Property Administration, PRC on August 04, 2020, which are incorporated herein by reference in their entireties.

## TECHNICAL FIELD

The present application relates to a crystalline form of an indolo heptamyl oxime analog for use as a PARP inhibitor and a method for preparing the same, and in particular to a crystalline form of a compound of formula (I) and a method for preparing the same.

## BACKGROUND

Ploy(ADP-ribose) polymerase (PARP) is a family of enzymes and can be used to catalyze the addition of ADP-ribose residues to a variety of target proteins. To date, a total of 18 subtypes have been identified and characterized. Despite the wide variety of enzymes in the PARP family, PARP-1 is responsible for more than 90% of ADP-ribosylation in cells, and thus PARP-1 inhibitors are the focus of PARP inhibitor research.

In the human living environment, human DNA is always damaged due to the influence of the natural environment (such as oxidative stress, radiotherapy and chemotherapy). PARP-1 is closely related to DNA repair and maintenance of genome function. Upon DNA damage, typically single strand break (SSB), PARP-1 first binds to the DNA break and is then activated, and as the structure of PARP1 enzyme changes, the enzyme begins to recruit NAD+ (coenzyme II) for the synthesis of poly(ADP)ribose, which at the same time serves as a signal for other repair enzymes such as DNA ligase and DNA polymerase β to function. This process of PARP-1 binding and activation is called base excision repair (BER), and contributes to the DNA amplification repair process. When PARP-1 is inhibited by a PARP inhibitor, a broken DNA cannot be repaired through SSB; instead, double strand break (DSB) is activated. The body repairs DSB mainly through two ways: homologous recombination (HR) and non-homologous end joining (NHEJ) of DNA, wherein the homologous recombination is the major way of DSB repair and features high repair reliability. BRCA1 and BRCA2 play important roles in homologous recombination (Nature, 2005, 913-917). Researches show that BRCA1/2 mutation is found in ovarian cancer, breast cancer and prostate cancer, and the PARP inhibitor is a good choice for BRCA1/2-deficient tumors. The PARP inhibitor can be used alone or in combination with chemotherapeutic drugs and radiotherapeutic drugs, thereby reducing the dosage and improving the efficacy. Based on this, a series of different types of compounds (J. Med. Chem. 2010, 4561) have been developed, and among these compounds, olaparib, rucaparib, niraparib (MK-4827) and talazoparib (BMN-673) have been approved for marketing. Nevertheless, as the indications of PARP inhibitors continue to expand, the application of PARP inhibitors is also deepening from treatment of tumor to that of stroke, myocardial ischemia, inflammation and diabetes. A very large number of clinical trials are currently in progress.

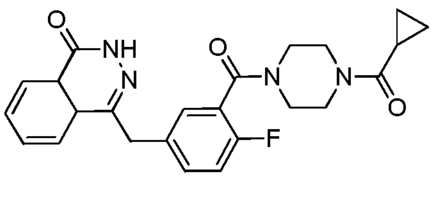

**Olaparib**

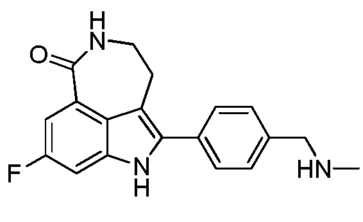

**Rucaparib**

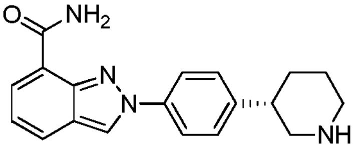

Niraparib

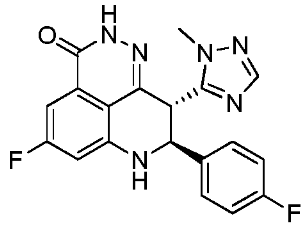

Talazoparib

Although efforts to develop PARP inhibitors for the treatment of cancer and other diseases is still ongoing, satisfactory treatment has not been found, and thus there is still a pressing need to develop new PARP inhibitors.

**BRIEF SUMMARY**

In one aspect, the present application provides a compound of formula (I),

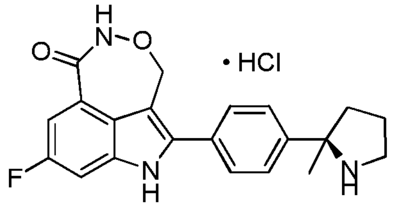

(I)

.

In another aspect, the present application provides a crystalline form of the compound of formula (I).

In another aspect, the present application provides a crystalline composition of the compound of formula (I), wherein the crystalline form of the compound of formula (I) described above accounts for 50% or more, preferably 75% or more, more preferably 90% or more, and most preferably 95% or more of the crystalline composition by weight. In another aspect, the present application provides a pharmaceutical composition comprising a therapeutically effective amount of the compound of formula (I) or the crystalline form thereof described above, or the crystalline composition of the compound of formula (I) described above; the pharmaceutical composition may comprise at least one pharmaceutically acceptable carrier or other excipient.

In another aspect, the present application provides use of the compound of formula (I) described above, the crystalline form of the compound of formula (I) described above, the crystalline composition of the compound of formula (I) described above, or the pharmaceutical composition described above in preparing a medicament for preventing or treating a PARP receptor-associated disorder.

In another aspect, the present application provides use of the compound of formula (I) described above, the crystalline form of the compound of formula (I) described above, the crystalline composition of the compound of formula (I) described above, or the pharmaceutical composition described above in preventing or treating a PARP receptor-associated disorder.

In another aspect, the present application provides a method for preventing or treating a PARP receptor-associated disorder, comprising administering to a mammal in need a therapeutically effective amount of the compound of formula (I) described above, the crystalline form of the compound of formula (I) described above, the crystalline composition of the compound of formula (I) described above, or the pharmaceutical composition described above. In another aspect, the present application provides the compound of formula (I) described above, the crystalline form of the compound of formula (I) described above, the crystalline composition of the compound of formula (I) described above, or the pharmaceutical composition described above for use as a PARP inhibitor.

In another aspect, the present application provides the compound of formula (I) described above, the crystalline form of the compound of formula (I) described above, the crystalline composition of the compound of formula (I) described above, or the pharmaceutical composition described above for use in preventing or treating a PARP receptor-associated disorder.

In another aspect, the present application provides a method for preparing the compound of formula (I) or the crystalline form thereof, wherein the method comprises: heating and stirring an amorphous form of the compound of formula (I) in an organic solvent, cooling for crystallization and filtering to give the crystalline form; the amorphous form

of the compound of formula (I) has an X-ray powder diffraction pattern using Cu Kα radiation shown in FIG. 1; in the method for preparing the crystalline form, the organic solvent is selected from the group consisting of acetone, tetrahydrofuran and ethyl acetate, and preferably acetone.

## SUMMARY

In one aspect, the present application provides a compound of formula (I),

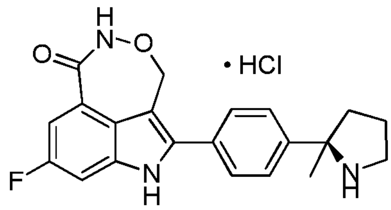

(I)

In another aspect, the present application further provides a crystalline form of the compound of formula (I).

In one embodiment of the present application, crystalline form A of the compound of formula (I) comprises 2, 3 or more characteristic peaks selected from the group consisting of the diffraction peaks at the following 2Θ angles in an X-ray powder diffraction pattern using Cu Kα radiation: 8.59 ± 0.20°, 16.00 ± 0.20°, 20.76 ± 0.20°, 25.14 ± 0.20° and 25.96 ± 0.20°.

In one embodiment of the present application, crystalline form A of the compound of formula (I) comprises 3, 4, 5, 6 or more characteristic peaks selected from the group consisting of the diffraction peaks at the following 2Θ angles in an X-ray powder diffraction pattern using Cu Kα radiation: 8.59 ± 0.20°, 11.74 ± 0.20°, 12.34 ± 0.20°, 16.00 ± 0.20°, 20.76 ± 0.20°, 25.14 ± 0.20° and 25.96 ± 0.20°.

In one embodiment of the present application, crystalline form A of the compound of formula (I) comprises 3, 4, 5, 6 or more characteristic peaks selected from the group consisting of the diffraction peaks at the following 2θ angles in an X-ray powder diffraction pattern using Cu Kα radiation: 8.59 ± 0.20°, 11.74 ± 0.20°, 12.56 ± 0.20°, 16.00 ± 0.20°, 20.76 ± 0.20°, 25.14 ± 0.20° and 25.96 ± 0.20°.

In one embodiment of the present application, crystalline form A of the compound of formula (I) comprises 3, 4, 5, 6 or more characteristic peaks selected from the group consisting of the diffraction peaks at the following 2Θ angles in an X-ray powder diffraction pattern using Cu Kα radiation: 8.59 ± 0.20°, 11.74 ± 0.20°, 12.34 ± 0.20°, 12.56 ± 0.20°, 16.00 ± 0.20°, 20.76 ± 0.20°, 25.14 ± 0.20° and 25.96 ± 0.20°.

In one embodiment of the present application, crystalline form A of the compound of formula (I) comprises 6, 7, 8, 9 or more characteristic peaks selected from the group consisting of the diffraction peaks at the following 2Θ angles in an X-ray powder diffraction pattern using Cu Kα radiation: 8.59 ± 0.20°, 11.74 ± 0.20°, 12.34 ± 0.20°, 12.56 ± 0.20°, 16.00 ± 0.20°, 17.20 ± 0.20°, 19.40 ± 0.20°, 20.76 ± 0.20°, 25.14 ± 0.20°, 25.96 ± 0.20°, 27.48 ± 0.20° and 31.68 ± 0.20°.

In one embodiment of the present application, crystalline form A of the compound of formula (I) comprises 9, 10, 11, 12 or more characteristic peaks selected from the group consisting of the diffraction peaks at the following 2Θ angles in an X-ray powder diffraction pattern using Cu Kα radiation: 8.59 ± 0.20°, 11.74 ± 0.20°, 12.34 ± 0.20°, 12.56 ± 0.20°, 13.32 ± 0.20°, 13.88 ± 0.20°, 15.38 ± 0.20°, 16.00 ± 0.20°, 17.20 ± 0.20°, 19.40 ± 0.20°, 20.76 ± 0.20°, 22.24 ± 0.20°, 22.56 ± 0.20°, 24.02 ± 0.20°, 25.14 ± 0.20°, 25.96 ± 0.20°, 27.48 ± 0.20°, 28.20 ± 0.20°, 29.32 ± 0.20°, 31.68 ± 0.20°, 31.98 ± 0.20° and 32.28 ± 0.20°.

In one embodiment of the present application, crystalline form A of the compound of formula (I) comprises 12, 13, 14, 15 or more characteristic peaks selected from the group consisting of the diffraction peaks at the following 2Θ angles in an X-ray powder diffraction pattern using Cu Kα radiation: 8.59 ± 0.20°, 11.74 ± 0.20°, 12.34 ± 0.20°, 12.56 ± 0.20°, 13.32 ± 0.20°, 13.88 ± 0.20°, 14.70 ± 0.20°, 15.38 ± 0.20°, 16.00 ± 0.20°, 17.20 ± 0.20°, 18.80 ± 0.20°, 19.40 ± 0.20°, 19.72 ± 0.20°, 20.76 ± 0.20°, 21.30 ± 0.20°, 21.73 ± 0.20°, 22.24 ± 0.20°, 22.56 ± 0.20°, 23.50 ± 0.20°, 24.02 ± 0.20°, 25.14 ± 0.20°, 25.96 ± 0.20°, 26.84 ± 0.20°, 27.48 ± 0.20°, 28.20 ± 0.20°, 29.32 ± 0.20°, 30.36 ± 0.20°, 31.68 ± 0.20°, 31.98 ± 0.20°, 32.28 ± 0.20°, 32.90 ± 0.20°, 33.59 ± 0.20°, 34.72 ± 0.20°, 35.22 ± 0.20°, 35.98 ± 0.20°, 36.58 ± 0.20° and 38.48 ± 0.20°.

In one embodiment of the present application, crystalline form A of the compound of formula (I) comprises diffraction peaks at the following 2Θ angles in an X-ray powder diffraction pattern using Cu Kα radiation: 8.59 ± 0.20°, 16.00 ± 0.20°, 20.76 ± 0.20°, 25.14 ± 0.20° and 25.96 ± 0.20°.

In one embodiment of the present application, crystalline form A of the compound of formula (I) comprises diffraction peaks at the following 2Θ angles in an X-ray powder diffraction pattern using Cu Kα radiation: 8.59 ± 0.20°, 11.74 ± 0.20°, 12.34 ± 0.20°, 16.00 ± 0.20°, 20.76 ± 0.20°, 25.14 ± 0.20° and 25.96 ± 0.20°.

In one embodiment of the present application, crystalline form A of the compound of formula (I) comprises diffraction peaks at the following 2Θ angles in an X-ray powder diffraction pattern using Cu Kα radiation: 8.59 ± 0.20°, 11.74 ± 0.20°, 12.56 ± 0.20°, 16.00 ± 0.20°, 20.76 ± 0.20°, 25.14 ± 0.20° and 25.96 ± 0.20°.

In one embodiment of the present application, crystalline form A of the compound of formula (I) comprises diffraction peaks at the following 2Θ angles in an X-ray powder diffraction pattern using Cu Kα radiation: 8.59 ± 0.20°, 11.74 ± 0.20°, 12.34 ± 0.20°, 12.56 ± 0.20°, 16.00 ± 0.20°, 20.76 ± 0.20°, 25.14 ± 0.20° and 25.96 ± 0.20°. In one embodiment of the present application, crystalline form A of the compound of formula (I) comprises diffraction peaks at the following 2Θ angles in an X-ray powder diffraction pattern using Cu Kα radiation: 8.59 ± 0.20°, 11.74 ± 0.20°, 12.34 ± 0.20°, 12.56 ± 0.20°, 16.00 ± 0.20°, 17.20 ± 0.20°, 19.40 ± 0.20°, 20.76 ± 0.20°, 25.14 ± 0.20°, 25.96 ± 0.20°, 27.48 ± 0.20° and 31.68 ± 0.20°.

In one embodiment of the present application, crystalline form A of the compound of formula (I) comprises diffraction peaks at the following 2Θ angles in an X-ray powder diffraction pattern using Cu Kα radiation: 8.59 ± 0.20°, 11.74 ± 0.20°, 12.34 ± 0.20°, 12.56 ± 0.20°, 13.32 ± 0.20°, 13.88 ± 0.20°, 15.38 ± 0.20°, 16.00 ± 0.20°, 17.20 ± 0.20°, 19.40 ± 0.20°, 20.76 ± 0.20°, 22.24 ± 0.20°, 22.56 ± 0.20°, 24.02 ± 0.20°, 25.14 ± 0.20°, 25.96 ± 0.20°, 27.48 ± 0.20°, 28.20 ± 0.20°, 29.32 ± 0.20°, 31.68 ± 0.20°, 31.98 ± 0.20° and 32.28 ± 0.20°.

In one embodiment of the present application, crystalline form A of the compound of formula (I) comprises diffraction peaks at the following 2Θ angles in an X-ray powder diffraction pattern using Cu Kα radiation: 8.59 ± 0.20°, 11.74 ± 0.20°, 12.34 ± 0.20°, 12.56 ± 0.20°, 13.32 ± 0.20°, 13.88 ± 0.20°, 14.70 ± 0.20°, 15.38 ± 0.20°, 16.00 ± 0.20°, 17.20 ± 0.20°, 18.80 ± 0.20°, 19.40 ± 0.20°, 19.72 ± 0.20°, 20.76 ± 0.20°, 21.30 ± 0.20°, 21.73 ± 0.20°, 22.24 ± 0.20°, 22.56 ± 0.20°, 23.50 ± 0.20°, 24.02 ± 0.20°, 25.14 ± 0.20°, 25.96 ± 0.20°, 26.84 ± 0.20°, 27.48 ± 0.20°, 28.20 ± 0.20°, 29.32 ± 0.20°, 30.36 ± 0.20°, 31.68 ± 0.20°, 31.98 ± 0.20°, 32.28 ± 0.20°, 32.90 ± 0.20°, 33.59 ± 0.20°, 34.72 ± 0.20°, 35.22 ± 0.20°, 35.98 ± 0.20°, 36.58 ± 0.20° and 38.48 ± 0.20°.

In one embodiment of the present application, crystalline form A of the compound of formula (I) has an X-ray powder diffraction pattern using Cu Kα radiation shown in FIG. 2.

In one embodiment of the present application, crystalline form A of the compound of formula (I) comprises characteristic peaks in an X-ray powder diffraction pattern using Cu Kα radiation with peak positions and relative intensities shown in Table 1.

**Table 1. XRPD pattern analysis data for crystalline form A of compound of formula (I)**

| No. | 2θ angle (± 0.2°) | Relative intensity (%) | No. | 2θ angle (± 0.2°) | Relative intensity (%) |
|---|---|---|---|---|---|
| 1 | 8.593 | 40.8 | 20 | 24.021 | 7.6 |
| 2 | 11.736 | 24.5 | 21 | 25.140 | 33.1 |
| 3 | 12.339 | 28.9 | 22 | 25.962 | 46.9 |
| 4 | 12.559 | 38.8 | 23 | 26.836 | 4.2 |
| 5 | 13.315 | 6.3 | 24 | 27.478 | 10.7 |
| 6 | 13.875 | 5.4 | 25 | 28.197 | 9.7 |
| 7 | 14.701 | 2.4 | 26 | 29.323 | 5.7 |
| 8 | 15.376 | 7.5 | 27 | 30.362 | 3.0 |
| 9 | 15.999 | 41.4 | 28 | 31.679 | 10.6 |
| 10 | 17.202 | 16.5 | 29 | 31.981 | 6.0 |
| 11 | 18.795 | 3.0 | 30 | 32.281 | 5.8 |
| 12 | 19.397 | 18.1 | 31 | 32.900 | 2.9 |
| 13 | 19.720 | 4.1 | 32 | 33.586 | 2.2 |
| 14 | 20.759 | 100.0 | 33 | 34.723 | 4.4 |
| 15 | 21.299 | 4.5 | 34 | 35.220 | 2.9 |
| 16 | 21.726 | 4.8 | 35 | 35.977 | 3.5 |
| 17 | 22.241 | 9.8 | 36 | 36.578 | 1.3 |

(continued)

| No. | 2θ angle (± 0.2°) | Relative intensity (%) | No. | 2θ angle (± 0.2°) | Relative intensity (%) |
|---|---|---|---|---|---|
| 18 | 22.562 | 7.7 | 37 | 38.479 | 2.7 |
| 19 | 23.498 | 4.8 | | | |

In one embodiment of the present application, crystalline form A of the compound of formula (I) has a starting point at 285.6 ± 5 °C of an exothermic peak in a differential scanning calorimetry curve.

In one embodiment of the present application, crystalline form A of the compound of formula (I) has a peak value at 288.6 ± 5 °C of an exothermic peak in a differential scanning calorimetry curve.

In one embodiment of the present application, crystalline form A of the compound of formula (I) has a differential scanning calorimetry (DSC) curve shown in FIG. 3.

In one embodiment of the present application, crystalline form A of the compound of formula (I) has a weight loss of 0.56% at 200.0 ± 5 °C in a thermogravimetric analysis curve.

In one embodiment of the present application, crystalline form A of the compound of formula (I) has a thermogravimetric analysis curve shown in FIG. 4.

The present application further provides crystalline form B of the compound of formula (I), comprising 1 or more characteristic peaks selected from the group consisting of the diffraction peaks at the following 2Θ angles in an X-ray powder diffraction pattern using Cu Kα radiation: 15.86 ± 0.20°, 21.62 ± 0.20° and 23.54 ± 0.20°.

The present application further provides crystalline form B of the compound of formula (I), comprising 2, 3, 4 or more characteristic peaks selected from the group consisting of the diffraction peaks at the following 2Θ angles in an X-ray powder diffraction pattern using Cu Kα radiation: 14.72 ± 0.20°, 15.86 ± 0.20°, 21.62 ± 0.20°, 23.14 ± 0.20°, 23.54 ± 0.20° and 26.28 ± 0.20°.

The present application further provides crystalline form B of the compound of formula (I), comprising 4, 5, 6, 7 or more characteristic peaks selected from the group consisting of the diffraction peaks at the following 2Θ angles in an X-ray powder diffraction pattern using Cu Kα radiation: 13.66 ± 0.20°, 14.72 ± 0.20°, 15.86 ± 0.20°, 18.30 ± 0.20°, 21.62 ± 0.20°, 23.14 ± 0.20°, 23.54 ± 0.20°, 25.80 ± 0.20°, 26.28 ± 0.20° and 27.96 ± 0.20°.

The present application further provides crystalline form B of the compound of formula (I), comprising 7, 8, 9, 10 or more characteristic peaks selected from the group consisting of the diffraction peaks at the following 2Θ angles in an X-ray powder diffraction pattern using Cu Kα radiation: 11.52 ± 0.20°, 13.66 ± 0.20°, 14.28 ± 0.20°, 14.72 ± 0.20°, 15.86 ± 0.20°, 16.84 ± 0.20°, 18.30 ± 0.20°, 18.72 ± 0.20°, 21.62 ± 0.20°, 23.14 ± 0.20°, 23.54 ± 0.20°, 24.48 ± 0.20°, 24.84 ± 0.20°, 25.80 ± 0.20°, 26.28 ± 0.20°, 27.96 ± 0.20°, 28.40 ± 0.20°, 28.84 ± 0.20°, 29.82 ± 0.20°, 30.22 ± 0.20°, 31.74 ± 0.20°, 32.04 ± 0.20°, 32.69 ± 0.20°, 33.90 ± 0.20°, 34.50 ± 0.20°, 35.02 ± 0.20°, 36.08 ± 0.20°, 36.94 ± 0.20°, 37.46 ± 0.20° and 37.92 ± 0.20°.

The present application further provides crystalline form B of the compound of formula (I), comprising diffraction peaks at the following 2Θ angles in an X-ray powder diffraction pattern using Cu Kα radiation: 15.86 ± 0.20°, 21.62 ± 0.20° and 23.54 ± 0.20°.

The present application further provides crystalline form B of the compound of formula (I), comprising diffraction peaks at the following 2Θ angles in an X-ray powder diffraction pattern using Cu Kα radiation: 14.72 ± 0.20°, 15.86 ± 0.20°, 21.62 ± 0.20°, 23.14 ± 0.20°, 23.54 ± 0.20° and 26.28 ± 0.20°.

The present application further provides crystalline form B of the compound of formula (I), comprising diffraction peaks at the following 2Θ angles in an X-ray powder diffraction pattern using Cu Kα radiation: 13.66 ± 0.20°, 14.72 ± 0.20°, 15.86 ± 0.20°, 18.30 ± 0.20°, 21.62 ± 0.20°, 23.14 ± 0.20°, 23.54 ± 0.20°, 25.80 ± 0.20°, 26.28 ± 0.20° and 27.96 ± 0.20°.

The present application further provides crystalline form B of the compound of formula (I), comprising diffraction peaks at the following 2Θ angles in an X-ray powder diffraction pattern using Cu Kα radiation: 11.52 ± 0.20°, 13.66 ± 0.20°, 14.28 ± 0.20°, 14.72 ± 0.20°, 15.86 ± 0.20°, 16.84 ± 0.20°, 18.30 ± 0.20°, 18.72 ± 0.20°, 21.62 ± 0.20°, 23.14 ± 0.20°, 23.54 ± 0.20°, 24.48 ± 0.20°, 24.84 ± 0.20°, 25.80 ± 0.20°, 26.28 ± 0.20°, 27.96 ± 0.20°, 28.40 ± 0.20°, 28.84 ± 0.20°, 29.82 ± 0.20°, 30.22 ± 0.20°, 31.74 ± 0.20°, 32.04 ± 0.20°, 32.69 ± 0.20°, 33.90 ± 0.20°, 34.50 ± 0.20°, 35.02 ± 0.20°, 36.08 ± 0.20°, 36.94 ± 0.20°, 37.46 ± 0.20° and 37.92 ± 0.20°.

In one embodiment of the present application, crystalline form B of the compound of formula (I) has an X-ray powder diffraction pattern using Cu Kα radiation shown in FIG. 5.

In one embodiment of the present application, crystalline form B of the compound of formula (I) comprises characteristic peaks in an X-ray powder diffraction pattern using Cu Kα radiation with peak positions and relative intensities shown in Table 2.

**Table 2. XRPD pattern analysis data for crystalline form B of compound of formula (I)**

| No. | 2θ angle (± 0.2°) | Relative intensity (%) | No. | 2θ angle (± 0.2°) | Relative intensity (%) |
|---|---|---|---|---|---|
| 1 | 11.519 | 5.5 | 16 | 27.962 | 18.8 |
| 2 | 13.659 | 10.9 | 17 | 28.402 | 5.4 |
| 3 | 14.276 | 7.4 | 18 | 28.844 | 3.3 |
| 4 | 14.721 | 22.4 | 19 | 29.819 | 3.8 |
| 5 | 15.860 | 63.9 | 20 | 30.220 | 5.7 |
| 6 | 16.840 | 5.2 | 21 | 31.738 | 7.4 |
| 7 | 18.299 | 11.6 | 22 | 32.040 | 2.8 |
| 8 | 18.715 | 6.7 | 23 | 32.686 | 2.3 |
| 9 | 21.621 | 100.0 | 24 | 33.899 | 4.0 |
| 10 | 23.141 | 33.0 | 25 | 34.500 | 6.4 |
| 11 | 23.541 | 64.0 | 26 | 35.021 | 6.1 |
| 12 | 24.483 | 5.7 | 27 | 36.082 | 5.3 |
| 13 | 24.838 | 3.8 | 28 | 36.941 | 6.0 |
| 14 | 25.801 | 17.7 | 29 | 37.459 | 3.7 |
| 15 | 26.281 | 22.7 | 30 | 37.918 | 2.0 |

In one embodiment of the present application, crystalline form B of the compound of formula (I) has a starting point at 287.4 ± 5 °C of an exothermic peak in a differential scanning calorimetry curve.

In one embodiment of the present application, crystalline form B of the compound of formula (I) has a peak value at 290.6 ± 5 °C of an exothermic peak in a differential scanning calorimetry curve.

In one embodiment of the present application, crystalline form B of the compound of formula (I) has a differential scanning calorimetry (DSC) curve shown in FIG. 6.

In one embodiment of the present application, crystalline form B of the compound of formula (I) has a weight loss of 0.70% at 200.0 ± 5 °C in a thermogravimetric analysis curve.

In one embodiment of the present application, crystalline form B of the compound of formula (I) has a thermogravimetric analysis curve shown in FIG. 7.

The present application further provides crystalline form C of the compound of formula (I), comprising 3, 4, 5, 6 or more characteristic peaks selected from the group consisting of the diffraction peaks at the following 2Θ angles in an X-ray powder diffraction pattern using Cu Kα radiation: 6.46 ± 0.20°, 18.78 ± 0.20°, 19.82 ± 0.20°, 20.98 ± 0.20°, 21.70 ± 0.20°, 23.30 ± 0.20° and 24.92 ± 0.20°.

The present application further provides crystalline form C of the compound of formula (I), comprising 6, 7, 8, 9 or more characteristic peaks selected from the group consisting of the diffraction peaks at the following 2Θ angles in an X-ray powder diffraction pattern using Cu Kα radiation: 6.46 ± 0.20°, 12.44 ± 0.20°, 18.78 ± 0.20°, 19.82 ± 0.20°, 20.22 ± 0.20°, 20.68 ± 0.20°, 20.98 ± 0.20°, 21.70 ± 0.20°, 22.32 ± 0.20°, 23.30 ± 0.20°, 24.08 ± 0.20°, 24.92 ± 0.20° and 27.82 ± 0.20°.

The present application further provides crystalline form C of the compound of formula (I), comprising 6, 7, 8, 9 or more characteristic peaks selected from the group consisting of the diffraction peaks at the following 2Θ angles in an X-ray powder diffraction pattern using Cu Kα radiation: 6.46 ± 0.20°, 12.44 ± 0.20°, 18.78 ± 0.20°, 19.82 ± 0.20°, 20.22 ± 0.20°, 20.68 ± 0.20°, 20.98 ± 0.20°, 21.70 ± 0.20°, 22.68 ± 0.20°, 23.30 ± 0.20°, 24.08 ± 0.20°, 24.92 ± 0.20° and 27.82 ± 0.20°.

The present application further provides crystalline form C of the compound of formula (I), comprising 6, 7, 8, 9 or more characteristic peaks selected from the group consisting of the diffraction peaks at the following 2Θ angles in an X-ray powder diffraction pattern using Cu Kα radiation: 6.46 ± 0.20°, 12.44 ± 0.20°, 18.78 ± 0.20°, 19.82 ± 0.20°, 20.22 ± 0.20°, 20.68 ± 0.20°, 20.98 ± 0.20°, 21.70 ± 0.20°, 22.32 ± 0.20°, 22.68 ± 0.20°, 23.30 ± 0.20°, 24.08 ± 0.20°, 24.92 ± 0.20° and 27.82 ± 0.20°.

The present application further provides crystalline form C of the compound of formula (I), comprising 9, 10, 11, 12 or more characteristic peaks selected from the group consisting of the diffraction peaks at the following 2Θ angles

in an X-ray powder diffraction pattern using Cu Kα radiation: 6.46 ± 0.20°, 9.94 ± 0.20°, 12.44 ± 0.20°, 16.32 ± 0.20°, 18.78 ± 0.20°, 19.82 ± 0.20°, 20.22 ± 0.20°, 20.68 ± 0.20°, 20.98 ± 0.20°, 21.70 ± 0.20°, 22.32 ± 0.20°, 22.68 ± 0.20°, 23.30 ± 0.20°, 24.08 ± 0.20°, 24.92 ± 0.20°, 26.14 ± 0.20°, 26.86 ± 0.20°, 27.82 ± 0.20°, 28.52 ± 0.20° and 29.34 ± 0.20°.

The present application further provides crystalline form C of the compound of formula (I), comprising 12, 13, 14, 15 or more characteristic peaks selected from the group consisting of the diffraction peaks at the following 2Θ angles in an X-ray powder diffraction pattern using Cu Kα radiation: 6.46 ± 0.20°, 9.59 ± 0.20°, 9.94 ± 0.20°, 10.58 ± 0.20°, 11.42 ± 0.20°, 12.44 ± 0.20°, 13.40 ± 0.20°, 14.86 ± 0.20°, 15.66 ± 0.20°, 16.32 ± 0.20°, 17.00 ± 0.20°, 17.54 ± 0.20°, 17.84 ± 0.20°, 18.78 ± 0.20°, 19.82 ± 0.20°, 20.22 ± 0.20°, 20.68 ± 0.20°, 20.98 ± 0.20°, 21.70 ± 0.20°, 22.32 ± 0.20°, 22.68 ± 0.20°, 23.30 ± 0.20°, 24.08 ± 0.20°, 24.92 ± 0.20°, 26.14 ± 0.20°, 26.86 ± 0.20°, 27.34 ± 0.20°, 27.82 ± 0.20°, 28.52 ± 0.20°, 29.34 ± 0.20°, 30.18 ± 0.20°, 31.36 ± 0.20° and 32.36 ± 0.20°.

The present application further provides crystalline form C of the compound of formula (I), comprising diffraction peaks at the following 2Θ angles in an X-ray powder diffraction pattern using Cu Kα radiation: 6.46 ± 0.20°, 18.78 ± 0.20°, 19.82 ± 0.20°, 20.98 ± 0.20°, 21.70 ± 0.20°, 23.30 ± 0.20° and 24.92 ± 0.20°.

The present application further provides crystalline form C of the compound of formula (I), comprising diffraction peaks at the following 2Θ angles in an X-ray powder diffraction pattern using Cu Kα radiation: 6.46 ± 0.20°, 12.44 ± 0.20°, 18.78 ± 0.20°, 19.82 ± 0.20°, 20.22 ± 0.20°, 20.68 ± 0.20°, 20.98 ± 0.20°, 21.70 ± 0.20°, 22.32 ± 0.20°, 23.30 ± 0.20°, 24.08 ± 0.20°, 24.92 ± 0.20° and 27.82 ± 0.20°.

The present application further provides crystalline form C of the compound of formula (I), comprising diffraction peaks at the following 2Θ angles in an X-ray powder diffraction pattern using Cu Kα radiation: 6.46 ± 0.20°, 12.44 ± 0.20°, 18.78 ± 0.20°, 19.82 ± 0.20°, 20.22 ± 0.20°, 20.68 ± 0.20°, 20.98 ± 0.20°, 21.70 ± 0.20°, 22.68 ± 0.20°, 23.30 ± 0.20°, 24.08 ± 0.20°, 24.92 ± 0.20° and 27.82 ± 0.20°.

The present application further provides crystalline form C of the compound of formula (I), comprising diffraction peaks at the following 2Θ angles in an X-ray powder diffraction pattern using Cu Kα radiation: 6.46 ± 0.20°, 12.44 ± 0.20°, 18.78 ± 0.20°, 19.82 ± 0.20°, 20.22 ± 0.20°, 20.68 ± 0.20°, 20.98 ± 0.20°, 21.70 ± 0.20°, 22.32 ± 0.20°, 22.68 ± 0.20°, 23.30 ± 0.20°, 24.08 ± 0.20°, 24.92 ± 0.20° and 27.82 ± 0.20°.

The present application further provides crystalline form C of the compound of formula (I), comprising diffraction peaks at the following 2Θ angles in an X-ray powder diffraction pattern using Cu Kα radiation: 6.46 ± 0.20°, 9.94 ± 0.20°, 12.44 ± 0.20°, 16.32 ± 0.20°, 18.78 ± 0.20°, 19.82 ± 0.20°, 20.22 ± 0.20°, 20.68 ± 0.20°, 20.98 ± 0.20°, 21.70 ± 0.20°, 22.32 ± 0.20°, 22.68 ± 0.20°, 23.30 ± 0.20°, 24.08 ± 0.20°, 24.92 ± 0.20°, 26.14 ± 0.20°, 26.86 ± 0.20°, 27.82 ± 0.20°, 28.52 ± 0.20° and 29.34 ± 0.20°.

The present application further provides crystalline form C of the compound of formula (I), comprising diffraction peaks at the following 2Θ angles in an X-ray powder diffraction pattern using Cu Kα radiation: 6.46 ± 0.20°, 9.59 ± 0.20°, 9.94 ± 0.20°, 10.58 ± 0.20°, 11.42 ± 0.20°, 12.44 ± 0.20°, 13.40 ± 0.20°, 14.86 ± 0.20°, 15.66 ± 0.20°, 16.32 ± 0.20°, 17.00 ± 0.20°, 17.54 ± 0.20°, 17.84 ± 0.20°, 18.78 ± 0.20°, 19.82 ± 0.20°, 20.22 ± 0.20°, 20.68 ± 0.20°, 20.98 ± 0.20°, 21.70 ± 0.20°, 22.32 ± 0.20°, 22.68 ± 0.20°, 23.30 ± 0.20°, 24.08 ± 0.20°, 24.92 ± 0.20°, 26.14 ± 0.20°, 26.86 ± 0.20°, 27.34 ± 0.20°, 27.82 ± 0.20°, 28.52 ± 0.20°, 29.34 ± 0.20°, 30.18 ± 0.20°, 31.36 ± 0.20° and 32.36 ± 0.20°.

In one embodiment of the present application, crystalline form C of the compound of formula (I) has an X-ray powder diffraction pattern using Cu Kα radiation shown in FIG. 8.

In one embodiment of the present application, crystalline form C of the compound of formula (I) comprises characteristic peaks in an X-ray powder diffraction pattern using Cu Kα radiation with peak positions and relative intensities shown in Table 3.

**Table 3. XRPD pattern analysis data for crystalline form C of compound of formula (I)**

| No. | 2θ angle (± 0.2°) | Relative intensity (%) | No. | 2θ angle (± 0.2°) | Relative intensity (%) |
|---|---|---|---|---|---|
| 1 | 6.460 | 100.0 | 18 | 20.979 | 52.1 |
| 2 | 9.593 | 13.4 | 19 | 21.702 | 50.5 |
| 3 | 9.941 | 20.9 | 20 | 22.321 | 40.0 |
| 4 | 10.582 | 17.7 | 21 | 22.683 | 45.0 |
| 5 | 11.422 | 17.3 | 22 | 23.300 | 51.0 |
| 6 | 12.438 | 34.1 | 23 | 24.080 | 37.4 |
| 7 | 13.402 | 6.3 | 24 | 24.921 | 71.2 |

(continued)

| No. | 2θ angle (± 0.2°) | Relative intensity (%) | No. | 2θ angle (± 0.2°) | Relative intensity (%) |
|---|---|---|---|---|---|
| 8 | 14.859 | 18.3 | 25 | 26.139 | 21.0 |
| 9 | 15.664 | 15.9 | 26 | 26.855 | 27.8 |
| 10 | 16.317 | 24.0 | 27 | 27.337 | 15.3 |
| 11 | 17.000 | 6.0 | 28 | 27.821 | 36.9 |
| 12 | 17.540 | 17.0 | 29 | 28.519 | 23.8 |
| 13 | 17.838 | 16.9 | 30 | 29.340 | 27.0 |
| 14 | 18.780 | 51.3 | 31 | 30.179 | 17.2 |
| 15 | 19.819 | 50.4 | 32 | 31.356 | 12.9 |
| 16 | 20.220 | 38.8 | 33 | 32.360 | 13.0 |
| 17 | 20.680 | 30.8 | | | |

In one embodiment of the present application, crystalline form C of the compound of formula (I) has a starting point at 261.6 ± 5 °C of an exothermic peak in a differential scanning calorimetry curve.

In one embodiment of the present application, crystalline form C of the compound of formula (I) has a peak value at 267.5 ± 5 °C of an exothermic peak in a differential scanning calorimetry curve.

In one embodiment of the present application, crystalline form C of the compound of formula (I) has a differential scanning calorimetry (DSC) curve shown in FIG. 9.

The present application further provides crystalline form D of the compound of formula (I), which is an amorphous form having an X-ray powder diffraction pattern using Cu Kα radiation shown in FIG. 1.

In one embodiment of the present application, crystalline form D of the compound of formula (I) is prepared from compound 1 in the presence of an organic solvent and hydrochloric acid.

In one embodiment of the present application, crystalline form D of the compound of formula (I) is prepared by stirring compound 1 in the presence of an organic solvent and hydrochloric acid, crystallizing and filtering.

In one embodiment of the present application, in the preparation of crystalline form D of the compound of formula (I), the organic solvent described above is selected from ethyl acetate.

In the present application, compound 1 has a structure as shown below:

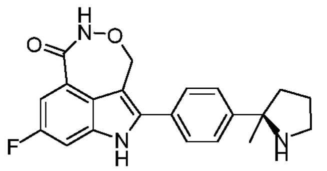

Compound 1

In one embodiment of the present application, crystalline form A of the compound of formula (I) is prepared from crystalline form D of the compound of formula (I) in an organic solvent.

In one embodiment of the present application, crystalline form A of the compound of formula (I) is prepared by heating and stirring crystalline form D of the compound of formula (I) in an organic solvent.

In one embodiment of the present application, crystalline form A of the compound of formula (I) is prepared by heating and stirring crystalline form D of the compound of formula (I) in an organic solvent, cooling for crystallization and filtering.

In one embodiment of the present application, in the preparation of crystalline form A of the compound of formula (I) described above, the organic solvent is selected from the group consisting of acetone, tetrahydrofuran and ethyl acetate, and preferably acetone.

In one embodiment of the present application, in the preparation of crystalline form A of the compound of formula (I) described above, the volume-to-molar ratio of the organic solvent to crystalline form D of the compound of formula (I) is (2-10) mL:1 mmol, and preferably (4-6) mL:1 mmol.

In one embodiment of the present application, in the preparation of crystalline form A of the compound of

formula (I) described above, the temperature for heating is 40-80 °C, and preferably 40-60 °C.

In one embodiment of the present application, in the preparation of crystalline form A of the compound of formula (I) described above, the temperature for heating is a reflux temperature of the organic solvent.

In one embodiment of the present application, in the preparation of crystalline form A of the compound of formula (I) described above, the time for stirring is 10-48 h, and preferably 16-48 h.

In one embodiment of the present application, in the preparation of crystalline form A of the compound of formula (I) described above, the crystallization is conducted by cooling to 25-35 °C, preferably 25-30 °C.

In another aspect, the present application provides crystalline form A of a compound of formula (I) prepared from crystalline form D of the compound of formula (I) in an organic solvent, wherein crystalline form A of the compound of formula (I) has the crystalline characteristics of crystalline form A described above.

In one embodiment of the present application, crystalline form A of the compound of formula (I) is prepared from crystalline form D of the compound of formula (I) in a solvent selected from one of or a mixed solvent of two or more of acetone, tetrahydrofuran and ethyl acetate.

In one embodiment of the present application, crystalline form A of the compound of formula (I) is prepared from crystalline form D of the compound of formula (I) in acetone.

In some embodiments of the present application, crystalline form B of the compound of formula (I) is prepared from crystalline form D of the compound of formula (I) in an organic solvent.

In one embodiment of the present application, crystalline form B of the compound of formula (I) is prepared by heating and stirring crystalline form D of the compound of formula (I) in an organic solvent.

In one embodiment of the present application, crystalline form B of the compound of formula (I) is prepared by heating and stirring crystalline form D of the compound of formula (I) in an organic solvent, cooling for crystallization and filtering.

In one embodiment of the present application, in the preparation of crystalline form B of the compound of formula (I) described above, the organic solvent is selected from acetonitrile.

In one embodiment of the present application, in the preparation of crystalline form B of the compound of formula (I) described above, the volume-to-molar ratio of the organic solvent to crystalline form D of the compound of formula (I) is (2-10) mL:1 mmol, and preferably (4-6) mL:1 mmol.

In one embodiment of the present application, in the preparation of crystalline form B of the compound of formula (I) described above, the temperature for heating is 30-80 °C, and preferably 40-60 °C.

In one embodiment of the present application, in the preparation of crystalline form B of the compound of formula (I) described above, the temperature for heating is a reflux temperature of the organic solvent.

In one embodiment of the present application, in the preparation of crystalline form B of the compound of formula (I) described above, the time for stirring is 10-48 h, and preferably 16-48 h.

In one embodiment of the present application, in the preparation of crystalline form B of the compound of formula (I) described above, the crystallization is conducted by cooling to 25-35 °C, preferably 25-30 °C.

In another aspect, the present application provides crystalline form B of a compound of formula (I) prepared from crystalline form D of the compound of formula (I) in an organic solvent, wherein crystalline form B of the compound of formula (I) has the crystalline characteristics of crystalline form B described above.

In one embodiment of the present application, crystalline form B of the compound of formula (I) is prepared from crystalline form D of the compound of formula (I) in acetonitrile.

In one embodiment of the present application, crystalline form C of the compound of formula (I) is prepared by dissociating crystalline form D of the compound of formula (I) in the presence of an organic solvent and a base, and then in the presence of hydrochloric acid.

In one embodiment of the present application, crystalline form C of the compound of formula (I) is prepared by dissociating crystalline form D of the compound of formula (I) in the presence of an organic solvent and a base, separating the phases, stirring in the presence of hydrochloric acid, crystallizing and filtering.

In one embodiment of the present application, in the preparation of crystalline form C of the compound of formula (I) described above, the organic solvent for the dissociation is selected from ethyl acetate.

In one embodiment of the present application, in the preparation of crystalline form C of the compound of formula (I) described above, the base for the dissociation is selected from the group consisting of sodium bicarbonate, sodium hydroxide, potassium hydroxide, potassium bicarbonate and sodium carbonate, and preferably sodium bicarbonate.

In one embodiment of the present application, in the preparation of crystalline form C of the compound of formula (I) described above, the base for the dissociation is in the form of a saturated aqueous solution of the base.

In one embodiment of the present application, in the preparation of crystalline form C of the compound of formula (I) described above, the molar:volume:volume ratio of crystalline form D of the compound of formula (I) to the organic solvent to the saturated aqueous solution of the base for the dissociation is 1 mmol:(12-20) mL:(4-10) mL, and preferably 1 mmol:(12-16) mL:(4-6) mL.

In one embodiment of the present application, in the preparation of crystalline form C of the compound of formula (I) described above, the dissociation is conducted under stirring for 8-12 minutes, and preferably 10 minutes.

In one embodiment of the present application, in the preparation of crystalline form C of the compound of formula (I) described above, the hydrochloric acid is in an organic solvent selected from the group consisting of ethyl acetate, chloroform, carbon tetrachloride and dioxane, and preferably ethyl acetate.

In one embodiment of the present application, in the preparation of crystalline form C of the compound of formula (I) described above, the equivalence ratio of crystalline form D of the compound of formula (I) to hydrochloric acid is 1:0.85.

In another aspect, the present application provides crystalline form C of a compound of formula (I) prepared from crystalline form D of the compound of formula (I) in an organic solvent, wherein crystalline form C of the compound of formula (I) has the crystalline characteristics of crystalline form C described above.

In one embodiment of the present application, crystalline form C of the compound of formula (I) is prepared from crystalline form D of the compound of formula (I) in ethyl acetate.

The hydrochloric acid described herein may be in a ready-to-use form prepared as a solution by introducing HCl gas into an organic solvent selected from the group consisting of ethyl acetate, chloroform, carbon tetrachloride and dioxane, and preferably ethyl acetate.

In another aspect, the present application provides a crystalline composition of the compound of formula (I), wherein the crystalline form of the compound of formula (I) described above accounts for 50% or more, preferably 75% or more, more preferably 90% or more, and most preferably 95% or more of the crystalline composition by weight. The crystalline composition may further comprise other crystalline or non-crystalline forms of the compound of formula (I) in small amounts.

In another aspect, the present application provides a pharmaceutical composition comprising a therapeutically effective amount of the compound of formula (I) described above, the crystalline form of the compound of formula (I) described above or the crystalline composition of the compound of formula (I) described above; the pharmaceutical composition may comprise at least one pharmaceutically acceptable carrier or other excipient.

In another aspect, the present application provides use of the compound of formula (I) described above, the crystalline form of the compound of formula (I) described above, the crystalline composition of the compound of formula (I) described above, or the pharmaceutical composition described above in preparing a medicament for preventing or treating a PARP receptor-associated disorder.

In another aspect, the present application provides use of the compound of formula (I) described above, the crystalline form of the compound of formula (I) described above, the crystalline composition of the compound of formula (I) described above, or the pharmaceutical composition described above in preventing or treating a PARP receptor-associated disorder.

In another aspect, the present application provides a method for preventing or treating a PARP receptor-associated disorder, comprising administering to a mammal in need a therapeutically effective amount of the compound of formula (I) described above, the crystalline form of the compound of formula (I) described above, the crystalline composition of the compound of formula (I) described above, or the pharmaceutical composition described above. In another aspect, the present application provides the compound of formula (I) described above, the crystalline form of the compound of formula (I) described above, the crystalline composition of the compound of formula (I) described above, or the pharmaceutical composition described above for use as a PARP inhibitor.

In another aspect, the present application provides the compound of formula (I) described above, the crystalline form of the compound of formula (I) described above, the crystalline composition of the compound of formula (I) described above, or the pharmaceutical composition described above for use in preventing or treating a PARP receptor-associated disorder.

In one embodiment of the present application, the mammal is a human.

In one embodiment of the present application, the PARP receptor-associated disorder is selected from the group consisting of a tumor or a cancer.

In one embodiment of the present application, the PARP receptor-associated disorder is selected from a breast cancer.

In another aspect, the present application provides a method for preparing the compound of formula (I) or the crystalline form thereof, wherein the method comprises: heating and stirring an amorphous form of the compound of formula (I) to an organic solvent, cooling for crystallization and filtering to give the crystalline form; the amorphous form of the compound of formula (I) has an X-ray powder diffraction pattern using Cu K$\alpha$ radiation shown in FIG. 1; in the method for preparing the crystalline form, the organic solvent is selected from the group consisting of acetone, tetrahydrofuran and ethyl acetate, and preferably acetone. Preferably, the crystalline form is crystalline form A.

In one embodiment of the present application, in the method for preparing the crystalline form, the volume-to-molar ratio of the organic solvent to the amorphous form of the compound of formula (I) is (2-10) mL:1 mmol, and preferably (4-6) mL:1 mmol.

In one embodiment of the present application, in the method for preparing the crystalline form, the amorphous form of the compound of formula (I) is prepared by stirring compound 1 in the presence of an organic solvent and hydrochloric acid, crystallizing and filtering; the organic solvent is selected from ethyl acetate; compound 1 has the following structure:

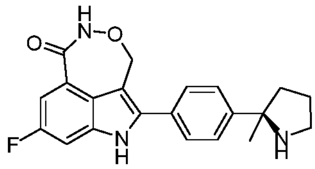

Compound 1

In the present application, the pharmaceutical composition can be formulated into a certain dosage form, and the route of administration is preferably oral administration, parenteral administration (including subcutaneous, intramuscular and intravenous administration), rectal administration, and the like. For example, suitable dosage forms for oral administration include tablets, capsules, granules, pulvises, pills, powders, pastilles, syrups or suspensions; suitable dosage forms for parenteral administration include aqueous or non-aqueous solutions or emulsions for injection; suitable dosage forms for rectal administration include suppositories with hydrophilic or hydrophobic carriers. The dosage forms described above may also be formulated as desired for rapid, delayed or modified release of the active ingredient.

The pharmaceutical composition disclosed herein can be manufactured using methods well known in the art, such as conventional mixing, dissolving, granulating, dragee-making, levigating, emulsifying, and lyophilizing.

The crystalline form described herein may be present in the form of a non-solvate or a solvate, for example, a hydrate.

In the present application, the X-ray powder diffraction pattern of a sample is determined under the following conditions: instrument: DX-2700BH X-ray diffractometer from (Dandong Haoyuan Instrument Co., Ltd., China); target: Cu:K$\alpha$; wavelength $\lambda$ = 1.54184 Å; range of 2$\theta$ angle: 3-40°; scattering slit: 1 mm; detector slit: 0.3 mm; anti-scatter slit: 1 mm; Cu-target tube voltage and current: 40 kV, 30 mA.

In the present application, the DSC spectrum is measured under the following conditions: instrument: METTLER TOLEDO DSC1 differential scanning calorimeter; temperature range: 40-350 °C; heating rate: 10 °C/min.

In the present application, the thermogravimetric analysis (TGA) is conducted under the following conditions: instrument: TA TGA550 thermogravimetric analyzer; flow rate: 40 mL/min; range of temperature: 40-500 °C; heating rate: 10 °C/min.

It should be noted that in the X-ray powder diffraction pattern, the position and relative intensity of a peak may vary due to measuring instruments, measuring methods/conditions, and other factors. For any specific crystalline form, the position of a peak may have an error, and the measurement of 2$\Theta$ has an error of $\pm$0.2°. Therefore, this error should be considered when determining each crystalline form, and crystalline forms within this margin of error are within the scope of the present application.

It should be noted that, for the same crystalline form, the position of an endothermic peak in the DSC pattern may vary due to measuring instruments, measuring methods/conditions, and other factors. For any specific crystalline form, the position of an endothermic peak may have an error of $\pm$5 °C. Therefore, this error should be considered when determining each crystalline form, and crystalline forms within this margin of error are within the scope of the present application.

It should be noted that, for the same crystalline form, the position of a weight loss temperature in the TGA pattern may vary due to measuring instruments, measuring methods/conditions, and other factors. For any specific crystalline form, the position of a weight loss temperature may have an error of $\pm$5 °C. Therefore, this error should be considered when determining each crystalline form, and crystalline forms within this margin of error are within the scope of the present application.

**Technical Effects**

Compound 1 disclosed herein has strong inhibitory activity against PARP1-kinase and excellent anti-proliferative activity against MDA-MB-436 cells with BRCA1 mutation, and meanwhile, it has no inhibitory activity against MDA-MB-231 cells with wild-type BRCA, showing that the compound disclosed herein is excellent in selectivity and safety. The compound 1 disclosed herein also has certain inhibitory effect against poly ADP-ribosylation. In addition, compound 1 disclosed herein has excellent pharmacokinetic properties as it is stable in metabolism *in vivo* and high in bioavailability. In general, the compound disclosed herein is not only excellent in activity and easy to synthesize, but also excellent in

pharmacokinetic properties.

The crystalline form of the compound of formula (I) described herein has stable properties, exhibits good stability under conditions of high humidity, high temperature, etc., and also exhibits good stability under long-term test conditions. The crystalline form of the compound of formula (I) described herein has advantages in terms of pharmaceutical activity, pharmacokinetics, bioavailability, stability, purity, ease of preparation, etc., and can meet the requirements of drug in terms of production, storage, transportation, formulation, etc.

**Definitions and Description**

Unless otherwise stated, the following terms and phrases used herein are intended to have the following meanings. A particular phrase or term, unless otherwise specifically defined, should not be considered as uncertain or unclear, but construed according to its common meaning. When referring to a trade name, it is intended to refer to its corresponding commercial product or its active ingredient.

"Mammal" includes human, domestic animals such as laboratory mammals and domestic pets (e.g., cat, dog, pig, cow, sheep, goat, horse, rabbit), and non-domesticated mammals such as wild mammals.

The term "pharmaceutical composition" refers to a formulation of the compound disclosed herein with a vehicle commonly recognized in the art for delivering a biologically active compound to a mammal, such as a human. The vehicle includes all pharmaceutically acceptable carriers for its use. The pharmaceutical composition facilitates administration of the compound to an organism.

The term "therapeutically effective amount" refers to an amount of a drug or an agent that is sufficient to provide the desired effect and is non-toxic. The determination of the effective amount varies from person to person. It depends on the age and general condition of a subject, as well as the particular active substance used. The appropriate effective amount in a case may be determined by those skilled in the art in the light of conventional tests.

The term "treat" or "treatment" means administering the compound or formulation described herein to ameliorate or eliminate a disease or one or more symptoms associated with the disease, including:

(i) inhibiting a disease or disease state, i.e., arresting its development; and
(ii) alleviating a disease or disease state, i.e., causing its regression.

The term "prevent" or "prevention" means administering the compound or formulation described herein to prevent a disease or one or more symptoms associated with the disease, including: preventing the occurrence of the disease or disease state in a mammal, particularly when such a mammal is predisposed to the disease state but has not yet been diagnosed with it.

In the present application, the term "pharmaceutically acceptable carrier" refers to a carrier, which is administered together with the active ingredient, and do not have a significant irritating effect on an organism or impair the biological activity and properties of the active compound. For additional information on carriers, reference may be made to *Remington: The Science and Practice of Pharmacy*, 21st Ed., Lippincott, Williams & Wilkins (2005), which is incorporated herein by reference.

In the present application, the term "room temperature" refers to 20 °C-30 °C.

In the present application, the term "EtOAc" stands for ethyl acetate.

The word "comprise", and variants thereof such as "comprises" or "comprising", or equivalents shall be interpreted in an open, non-exclusive sense, i.e., "includes but is not limit to", indicating that in addition to the listed elements, components and procedures, other unspecified elements, components and procedures may also be encompassed. Unless otherwise specified clearly herein, singular terms encompass plural terms, and vice versa.

Unless otherwise indicated herein, parameter values (including 2Θ values, reaction conditions) are to be construed as modified by the term "about" to reflect the measurement error and the like existing in the values, e.g., there is an error of ±5% relative to the given value.

All patents, patent applications and other identified publications are explicitly incorporated herein by reference for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

**BRIEF DESCRIPTION OF THE DRAWINGS**

FIG. 1 is an XRPD pattern of crystalline form D of the compound of formula (I).

FIG. 2 is an XRPD pattern of crystalline form A of the compound of formula (I) prepared by Method I in Example 2.

FIG. 3 is a DSC pattern of crystalline form A of the compound of formula (I).

FIG. 4 is a TGA pattern of crystalline form A of the compound of formula (I).

FIG. 5 is an XRPD pattern of crystalline form B of the compound of formula (I).

FIG. 6 is a DSC pattern of crystalline form B of the compound of formula (I).

FIG. 7 is a TGA pattern of crystalline form B of the compound of formula (I).

FIG. 8 is an XRPD pattern of crystalline form C of the compound of formula (I).

FIG. 9 is a DSC pattern of crystalline form C of the compound of formula (I).

FIG. 10 is an ellipsoid plot of the three-dimensional structure of crystalline form A of the compound of formula (I).

## DETAILED DESCRIPTION

The present application is described in detail below by way of examples. However, this is by no means disadvantageously limiting the scope of the present application. The compounds disclosed herein can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application. It will be apparent to those skilled in the art that various changes and modifications can be made to the specific embodiments without departing from the spirit and scope of the present application.

### Example 1. Preparation of compound 1

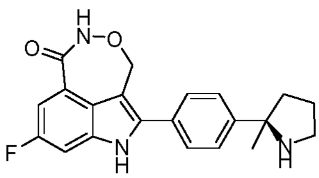

Compound 1

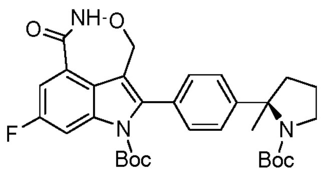

Compound 1_AA

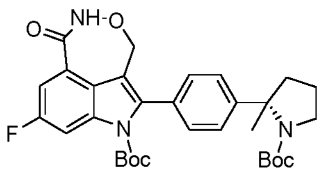

Compound 1_BB

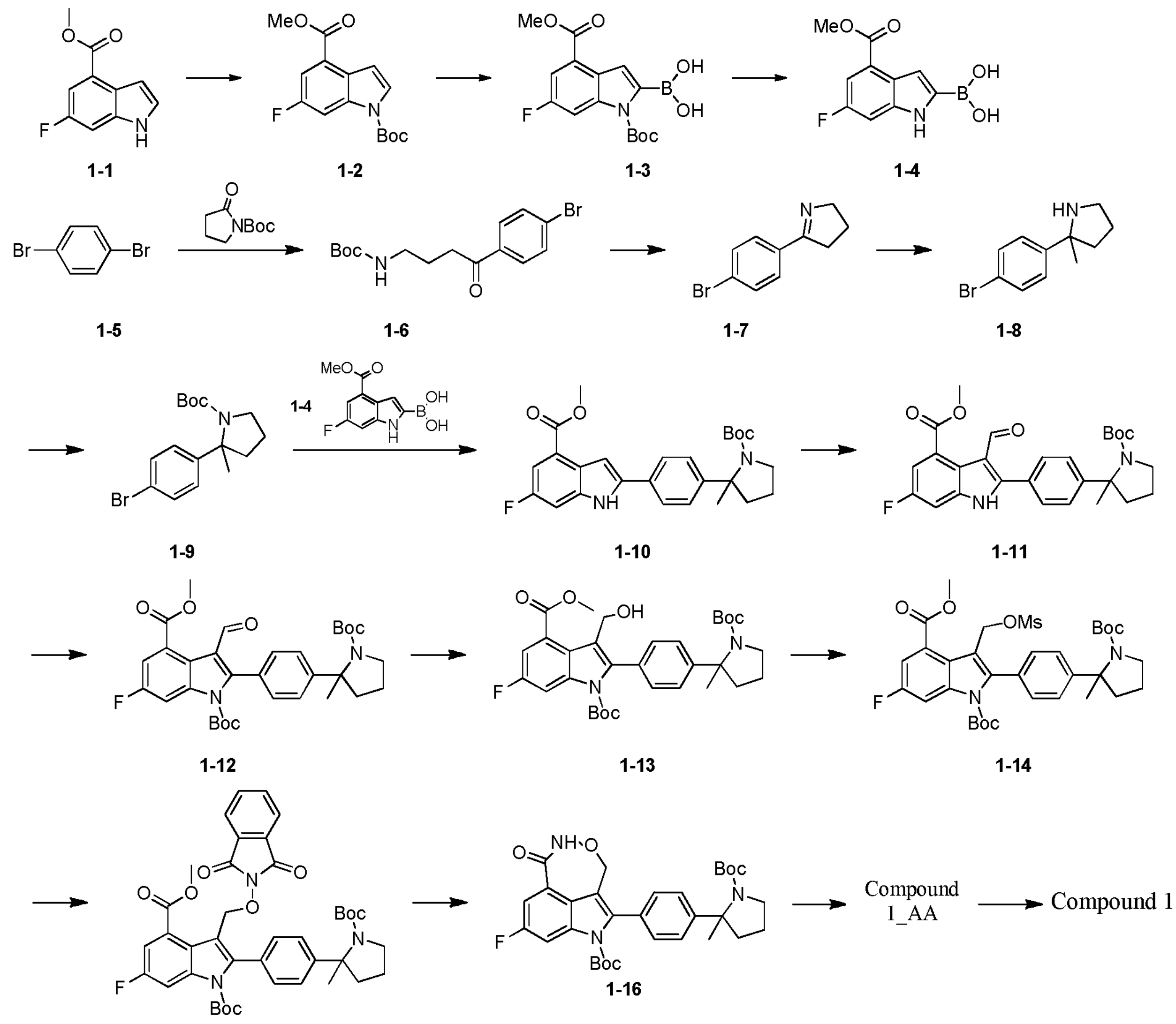


1-1 1-2 1-3 1-4

1-5 1-6 1-7 1-8

1-9 1-10 1-11

1-12 1-13 1-14

1-15 1-16

Step A: 1-1 (25 g, 129.42 mmol) was dissolved in dichloromethane (250 mL), and triethylamine (26.19 g, 258.83 mmol), 4-dimethylaminopyridine (3.16 g, 25.88 mmol), di-*tert*-butyl dicarbonate (31.07 g, 142.36 mmol) were added at 0 °C. The reaction system was stirred at 25 °C for 2 h. The reaction system was washed with saturated aqueous ammonium chloride solution (80 mL × 3) and saturated brine (50 mL × 2), dried over anhydrous sodium sulfate and filtered. The filtrate was concentrated at reduced pressure to give 1-2.

Step B: Diisopropylamine (13.80 g, 136.38 mmol) was dissolved in tetrahydrofuran (60 mL), and *n*-butyllithium (2.5 M, 47.73 mL) was added dropwise to the reaction system at -78 °C in nitrogen atmosphere. The dropwise addition was completed within half an hour. The reaction solution was stirred at 0 °C for half an hour, and then added dropwise to another three-necked flask containing a solution of 1-2 (25 g, 285.14 mmol) and triisopropyl borate (24.05 g, 127.86 mmol) in tetrahydrofuran (200 mL) at 0 °C in nitrogen atmosphere. The dropwise addition was completed quickly at 0 °C. The reaction solution was stirred at 0 °C for 1 h. Acetic acid solution (50 mL) was then added to the reaction system to quench the reaction. The resulting solution was diluted with water (60 mL) and extracted with ethyl acetate (60 mL × 3). The organic phases were combined, washed with saturated aqueous ammonium chloride solution (50 mL × 3) and saturated brine (40 mL × 2), dried over anhydrous sodium sulfate and filtered. The filtrate was concentrated at reduced pressure to give a crude product. The crude product was slurried with acetonitrile (100 mL) and aqueous solution (300 mL), and the filter cake was dried with an oil pump to give 1-3.

Step C: 1-3 (45 g, 133.49 mmol) was added to a solution of trifluoroacetic acid (200 mL) at 0 °C in three portions, and the reaction system was stirred at 0 °C for 1 h in nitrogen atmosphere. The reaction solution was then poured into ice water (300 mL) to precipitate a solid, and a filter cake was obtained and then concentrated at reduced pressure with an oil pump to give 1-4.

Step D: 1-5 (25 g, 105.98 mmol) was dissolved in tetrahydrofuran (100.00 mL) and the resulting mixture was

added dropwise to a three-necked flask containing magnesium chips (2.58 g, 105.98 mmol) and iodine (489.05 mg, 1.93 μmol) at 70 °C in nitrogen atmosphere. The dropwise addition was completed within half an hour. The reaction solution was stirred at 70 °C for 1 h, and then cooled to 20 °C. The reaction solution was added dropwise into another three-necked flask containing a solution of *tert*-butyl 2-oxopyrrolidine-1-carboxylate (17.84 g, 96.34 mmol) in tetrahydrofuran (150 mL) at -70 °C in nitrogen atmosphere. The dropwise addition was completed within half an hour. The reaction solution was stirred at -70 °C for 2 h, then warmed to 10 °C and stirred for 1 h. Saturated aqueous ammonium chloride solution (60 mL) was added to the reaction system to quench the reaction. The resulting mixture was extracted with ethyl acetate (60 mL × 3). The organic phases were combined, washed with saturated brine (50 mL), dried over anhydrous sodium sulfate and filtered. The filtrate was concentrated at reduced pressure. The residue was purified by silica gel column to give 1-6.

Step E: 1-6 (50 g, 146.10 mmol) was added to trifluoroacetic acid (250 mL) at 0 °C. The reaction system was stirred at 15 °C for 12 h. The reaction solution was adjusted to pH *14* with 40% aqueous sodium hydroxide solution and a yellow solid was precipitated. The resulting mixture was filtered, and the filter cake was washed with a small amount of water and subjected to rotary evaporation to give 1-7.

Step F: 1-7 (15 g, 66.94 mmol) was dissolved in tetrahydrofuran (150 mL). The reaction system was cooled to - 78 °C in nitrogen atmosphere, and boron trifluoride diethyl etherate (19 g, 133.87 mmol) was added dropwise to the reaction system. The dropwise addition was completed within half an hour. The reaction system was stirred at - 78 °C for half an hour. Methyllithium solution (1.6 M, 83.67 mL) was then added dropwise to the reaction system. The reaction system was slowly warmed to 78 °C and stirred for 19.5 h. The reaction system was cooled to room temperature, and saturated aqueous sodium bicarbonate solution (100 mL) was added to quench the reaction. Water (30 mL) was then added and the resulting mixture was extracted with ethyl acetate (80 mL × 3). The organic phases were combined, washed with saturated brine (30 mL × 2), dried over anhydrous sodium sulfate and filtered. The filtrate was concentrated at reduced pressure to give 1-8.

Step G: 1-8 (16 g, 66.63 mmol) was dissolved in dichloromethane (150 mL), and triethylamine (20.23 g, 199.88 mmol) was added at 0 °C, followed by the addition of di-*tert*-butyl dicarbonate (29.08 g, 133.26 mmol). The reaction system was stirred at 15 °C for 1 h. The reaction system was concentrated to dryness by rotary evaporation at reduced pressure. Saturated aqueous ammonium chloride solution (60 mL) was added and the resulting mixture was extracted with ethyl acetate (80 mL × 3). The organic phases were combined, washed with saturated brine (30 mL × 3), dried over anhydrous sodium sulfate and filtered. The filtrate was concentrated at reduced pressure to give 1-9.

Step H: 1-9 (9 g, 26.45 mmol) and 1-4 (7.52 g, 31.74 mmol) were dissolved in ethylene glycol dimethyl ether (100 mL) and water (20 mL), and sodium bicarbonate (6.67 g, 79.35 mmol) and [1,1-bis(di-*tert*-butylphosphino)ferrocene] palladium dichloride (1.72 g, 2.65 mmol) were added. The reaction system was purged with nitrogen three times and stirred at 80 °C for 12 h in nitrogen atmosphere. The reaction system was concentrated to dryness by rotary evaporation at reduced pressure. Saturated brine (60 mL) was added and the resulting mixture was extracted with ethyl acetate (60 mL × 3). The organic phases were combined, washed with saturated brine (50 mL), dried over anhydrous sodium sulfate and filtered. The filtrate was concentrated at reduced pressure to give a residue. The residue was purified by silica gel column to give 1-10.

Step I: Oxalyl chloride (5.61 g, 44.20 mmol) was added to dichloromethane (150 mL), and *N,N*-dimethylformamide (4.85 g, 66.30 mmol) was slowly and dropwise added at 0 °C in nitrogen atmosphere. The reaction system was stirred at 0 °C for 15 min. 1-10 (10 g, 22.10 mmol) was then dissolved in dichloromethane (50 mL) and added to the reaction system at 0 °C. The reaction system was stirred at 15 °C for 0.5 h. 10% aqueous ammonium acetate solution (100 mL) and tetrahydrofuran (100 mL) were added to the reaction system to quench the reaction and the resulting mixture was extracted with ethyl acetate (45 mL × 2). The organic phases were combined, washed with saturated aqueous ammonium chloride solution (50 mL × 3) and saturated brine (50 mL × 3), dried over anhydrous sodium sulfate and filtered. The filtrate was concentrated at reduced pressure to give 1-11.

Step J: 1-11 (10.62 g, 22.10 mmol) was dissolved in dichloromethane (80 mL), and triethylamine (6.71 g, 66.30 mmol), di-tert-butyl dicarbonate (9.65 g, 44.20 mmol) and 4-dimethylaminopyridine (810.01 mg, 6.63 mmol) were added at 0 °C. The reaction system was stirred at 15 °C for 1 h. The reaction system was concentrated to dryness by rotary evaporation at reduced pressure. Saturated aqueous ammonium chloride solution (60 mL) was added and the resulting mixture was extracted with ethyl acetate (60 mL × 3). The organic phases were combined, washed with saturated brine (30 mL × 3), dried over anhydrous sodium sulfate and filtered. The filtrate was concentrated at reduced pressure to give 1-12.

Step K: 1-12 (12.75 g, 21.96 mmol) was dissolved in tetrahydrofuran (100 mL) and methanol (25 mL). The reaction system was cooled to 0 °C, and sodium borohydride (1.25 g, 32.94 mmol) was added. The reaction system was stirred at 0 °C for 40 min. Saturated aqueous ammonium chloride solution (80 mL) was added to the reaction system to quench the reaction. The resulting mixture was extracted with ethyl acetate (80 mL × 2). The organic phases were combined, washed with water (50 mL), dried over anhydrous sodium sulfate and filtered. The filtrate was concentrated at reduced pressure to give 1-13.

Step L: 1-13 (12.79 g, 21.95 mmol) was dissolved in dichloromethane (150 mL), and triethylamine (4.44 g, 43.90 mmol) was added, followed by the addition of methanesulfonyl chloride (3.02 g, 26.34 mmol) at 0 °C in nitrogen atmosphere. The reaction system was stirred at 0 °C for 1 h. The reaction system was concentrated to dryness by rotary evaporation at reduced pressure and ethyl acetate (80 mL) was added. The organic phase was washed with saturated aqueous ammonium chloride solution (30 mL × 2) and saturated brine (20 mL × 2), dried over anhydrous sodium sulfate and filtered. The filtrate was concentrated at reduced pressure to give 1-14.

Step M: 1-14 (14.45 g, 21.87 mmol) was dissolved in *N,N*-dimethylformamide (150 mL), and sodium carbonate (4.64 g, 43.74 mmol) and *N*-hydroxyphthalimide (5.35 g, 32.80 mmol) were added. The reaction system was stirred at 65 °C for 12 h. An aqueous solution (60 mL) was added to the reaction system and the resulting mixture was extracted with ethyl acetate (80 mL × 2). The organic phase was washed with saturated aqueous ammonium chloride solution (50 mL × 3) and saturated brine (50 mL × 3), dried over anhydrous sodium sulfate and filtered. The filtrate was concentrated at reduced pressure to give a residue. The residue was purified by silica gel column to give 1-15. Step N: 1-15 (15 g, 20.61 mmol) was dissolved in methanol (200 mL), and 98% hydrazine hydrate (3.10 g, 61.83 mmol) was added. The reaction system was stirred at 65 °C for 2 h in nitrogen atmosphere. The reaction system was filtered, and the filtrate was concentrated at reduced pressure to give a residue. The residue was purified by silica gel column to give 1-16.

Step O: Compound 1-16 was separated by chiral HPLC column (separation column AD-H (250 mm × 30 mm, 5 μm); mobile phase: 0.1% ammonia in isopropanol; elution gradient 25%-25%, 2.7 min; 400 min) to give **1_AA** (**retention time** = **2.161 min**, ee (enantiomeric excess) value: 100%) and **1_BB** (**retention time** = **2.353 min**, ee (enantiomeric excess) value: 97%).

Method for measuring ee (enantiomeric excess) value: analytical column: Amycoat 50 mm × 4.6 mm I.D., 3 μm; mobile phase: 10%-20% isopropanol (0.05% diethylamine) in $CO_2$; flow rate: 3 mL/min; wavelength: 220 nm. Step P: **1_AA** was deprotected with trifluoroacetic acid to give **compound 1** (time = 3.461 min, ee (enantiomeric excess) value: 98%).

Method for measuring ee (enantiomeric excess) value: separation column: Chiralcel Cellucoat 50 mm × 4.6 mm I.D., 3 μm; mobile phase: 10%-40% isopropanol (0.05% diethylamine) in $CO_2$; flow rate: 3 mL/min; wavelength: 220 nm.

**Compound 1: $^1$H NMR (400 MHz, deuterated dimethyl sulfoxide)** δ = 12.00 (br s, 1H), 11.30 (br s, 1H), 8.23 (br s, 1H), 7.62 (br d, *J* = 7.75 Hz, 4H), 7.45 (br d, *J* = 9.13 Hz, 2H), 5.44 (br d, *J* = 14.51 Hz, 1H), 5.14-5.31 (m, 1H), 2.99-3.42 (m, 2H), 1.89-2.23 (m, 4H), 1.53 (br s, 3H).

**Example 2. Preparation of crystalline forms A, B, C and D of compound of formula (I)**

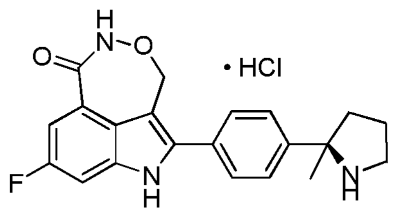

(I)

**Preparation of crystalline form D:**

Step 1: HCl gas was introduced into EtOAc (100 mL) at -40 to -20 °C to prepare a solution of HCl/EtOAc (hydrogen chloride in ethyl acetate, 7 mol, 100 mL).

Step 2: Compound **1_AA** (10 g, 17.68 mmol) was added to the above solution in portions at -20 to -10 °C. The cold bath was removed after the addition. The reaction solution was naturally warmed to about 20 °C and stirred for 3 h. A large amount of solid was precipitated.

Step 3: The mixture was filtered and washed three times with ethyl acetate (30 mL × 3). The solid was collected and dried in an oven for 16 h to give crystalline form D of the compound of formula (I). $^1$H NMR (400 MHz, DMSO-$d_6$) δ 12.15 (br s, 1H), 11.30 (s, 1H), 10.45 (br s, 1H), 9.29 (br s, 1H), 7.61-7.75 (m, 4H), 7.39-7.51 (m, 2H), 5.45 (br d, J = 14.79 Hz, 1H), 5.23 (br dd, J = 7.40, 14.73 Hz, 1H), 3.29-3.36 (m, 2H), 2.34-2.45 (m, 1H), 2.14-2.30 (m, 2H), 2.05 (br d, J = 4.16 Hz, 1H), 1.60 (s, 3H).

### Preparation of crystalline form A:

Method I:

Crystalline form D (1 g, 2.49 mmol) of the compound of formula (I) was added to 10 mL of acetone. The resulting mixture was stirred at reflux for 16 h, cooled to about 30 °C and filtered. The solid was collected and dried at 35-40 °C to give crystalline form A of the compound of formula (I).
$^1$HNMR (400 MHz, DMSO-$d_6$) $\delta$ = 12.24-12.06 (m, 1H), 11.41-11.22 (m, 1H), 10.39 (br s, 1H), 9.50-8.97 (m, 1H), 7.79-7.59 (m, 4H), 7.55-7.33 (m, 2H), 5.56-5.37 (m, 1H), 5.31-5.15 (m, 1H), 3.50-3.42 (m, 1H), 3.36-3.29 (m, 1H), 2.45-2.34 (m, 1H), 2.15 (br s, 2H), 2.14-2.01 (m, 1H), 1.61 (s, 3H).

Method II:
Crystalline form D of the compound of formula (I) (200 mg, 0.50 mmol) was added to 2 mL of ethyl acetate, and the resulting mixture was stirred at 40 °C for 48 h and filtered. The solid was collected and dried at 35-40 °C to give crystalline form A of the compound of formula (I).
Method III:
Crystalline form D of the compound of formula (I) (200 mg, 0.50 mmol) was added to 2 mL of tetrahydrofuran, and the resulting mixture was stirred at 40 °C for 48 h and filtered. The solid was collected and dried at 35-40 °C to give crystalline form A of the compound of formula (I).

### Preparation of crystalline form B:

Crystalline form D of the compound of formula (I) (1 g, 2.49 mmol) was added to 10 mL of acetonitrile, and the resulting mixture was stirred at reflux for 16 h, cooled to about 30 °C and filtered. The solid was collected and dried to give crystalline form B of the compound of formula (I).
$^1$H NMR (400 MHz, DMSO-$d_6$) $\delta$ = 12.14 (d, J = 3.1 Hz, 1H), 11.30 (s, 1H), 10.47 (br s, 1H), 9.27 (br d, J = 9.5 Hz, 1H), 7.76-7.60 (m, 4H), 7.45 (dt, J = 2.3, 9.5 Hz, 2H), 5.56-5.38 (m, 1H), 5.22 (dd, J = 7.9, 14.8 Hz, 1H), 3.49-3.40 (m, 1H), 3.34 (br d, J = 7.0 Hz, 1H), 2.38 (br d, J = 8.0 Hz, 1H), 2.29-2.13 (m, 2H), 2.08 (s, 1H), 2.07 (s, 1H), 2.07-1.99 (m, 1H), 1.59 (s, 3H).

### Preparation of crystalline form C:

Crystalline form D of the compound of formula (I) (10 g, 2.49 mmol) was added to 40 mL of ethyl acetate, and then saturated $NaHCO_3$ solution (15 mL) was added. The resulting mixture was stirred for 10 min, and the phases were separated. The solid was dried and then added into a suitable reaction flask and cooled to -20 °C. Hydrogen chloride in ethyl acetate (0.5 M, 4.23 mL, 0.85 equivalent) was then slowly and dropwise added. After the dropwise addition, the resulting mixture was naturally cooled to room temperature, reacted for 2.5 h and filtered. The solid was collected and dried to give crystalline form C of the compound of formula (I).
$^1$H NMR (400 MHz, DMSO-$d_6$) $\delta$ 12.11-12.19 (m, 1H), 11.30 (s, 1H), 10.41 (br s, 1H), 9.20-9.35 (m, 1H), 7.63-7.72 (m, 4H), 7.42-7.50 (m, 2H), 5.45 (br d, J=15.28 Hz, 1H), 5.23 (dd, J=7.15, 14.86 Hz, 1H), 3.44-3.66 (m, 1H), 3.31-3.36 (m, 1H), 2.33-2.45 (m, 1H), 2.14-2.29 (m, 2H), 2.00-2.11 (m, 1H), 1.60 (s, 3H).

### Experimental Example 1. Stability test of crystalline form A and crystalline form B of compound of formula (I)

1. Preliminary stability examination

Examination conditions: The stability of crystalline form A and crystalline form B was simultaneously examined at 60 °C and 75% humidity. The results are as follows:

| **Crystalline form** | **Preliminary stability examination** (content, %) | |
|---|---|---|
| | **60°C 75% RH** | |
| | **Day 0** | **Day 10** |
| Crystalline form A | 99.73 | 99.72 |
| Crystalline form B | 99.09 | 98.93 |

2. Accelerated test

Examination conditions: Samples of crystalline form A of the compound of formula (I) were packed into double-layer pharmaceutical low-density polyethylene bags. Each layer of the pharmaceutical low-density polyethylene bag was sealed, then the bags were put into aluminum foil bags, and the aluminum foil bags were heat-sealed. Accelerated test was conducted at 40 °C ± 2 °C and 75 ± 5% RH. The results are as follows:

| Detection items | Initial values | 40°C±2°C /75±5%RH | | | |
|---|---|---|---|---|---|
| | | Month 1 | Month 2 | Month 3 | Month 6 |
| Appearance | Earthy yellow powder | Earthy yellow powder | Earthy yellow powder | Earthy yellow powder | Earthy yellow powder |
| Content | 98.0% | 96.2% | 96.9% | 96.9% | 97.5% |
| Total impurities | 0.45% | 0.75% | 0.78% | 0.62% | 0.93% |
| Isomer | Not detectable | Not detectable | Not detectable | Not detectable | Not detectable |
| Water content | 2.07% | 2.50% | 2.26% | 1.89% | 2.14% |
| XRPD | As a reference pattern | Not available | Not available | Consistent with the pattern on day 0 | Consistent with the pattern on day 0 |

Conclusion: Crystalline form A and crystalline form B of the compound of formula (I) have good stability.

**Experimental Example 2. PARP-1 enzymatic assay**

**Materials:** test compounds; HT Universal Chemiluminescent PARP Assay kit (purchased from TREVIGEN); PBS (phosphate buffer saline, purchased from Wisent); Triton X-100 (purchased from Macklin); Envision Multimode Plate Reader (PerkinElmer).

Composition of HT Universal Chemiluminescent PARP Assay kit

| Composition | Volume | Storage conditions |
|---|---|---|
| PARP-HSA, 10 U/μL | 50 μL | -20°C |
| 20X PARP Buffer (2 vials) | 2×500 μL | -80°C |
| 10X PARP Cocktail | 300 μl | -80°C |
| Histone-Coated White Strip Wells | 96 wells | 4°C |
| 10X Strep-Diluent | 2 ml | 4°C |
| Strep-HRP | 30 μl | 4°C |
| PeroxyGlow™ A | 6 mL | 4°C |
| PeroxyGlow™ B | 6 mL | 4°C |
| 10X Activated DNA | 300 μL | -20°C |

**Procedures:**

(I) Preparation of reagents:

1. Washing solution: Triton X-100 was added to 1× PBS with a final concentration of Triton X-100 of 0.1%.
2. 1× PARP buffer: The 20× PARP buffer in the kit was 20-fold diluted with water to prepare a 1× PARP buffer, which was used to prepare the compounds, enzyme solutions and substrate solutions.
3. 1× Strep-Diluent solution: The 10× Strep-diluent in the kit was 10-fold diluted with water to prepare a 1× Strep-diluent solution.

(II) Preparation of test compounds:
The test compounds were diluted from 200 μM to 2.56 nM. The DMSO concentration was 100%. 2 μL of each of the inhibitors at various concentration gradients was added to a compound intermediate plate, and 38 μL of the 1× PARP buffer was then added. The two were mixed well for use, and the DMSO concentration was 5% at this time.
(III) Procedures:

a) The 1× PARP buffer was added to a test plate at 50 μL per well, and the test plate was incubated at 25 °C for 30 min.

b) After the incubation was completed, the liquid in the test plate was discarded, and each of the compounds at various concentration gradients was pipetted from the compound intermediate plate and added into the test plate at 10 μL per well. The samples were in duplicate.

c) The enzyme solution (0.5 IU) was added to the test plate at 15 μL per well. The compound and enzyme were co-incubated at 25 °C for 10 min.

d) After the incubation was completed, 25 μL of the 1 × PARP Cocktail (comprising 2.5 μL of 10× PARP Cocktail, 2.5 μL of 10× Activated DNA and 20 μL of 1 × PARP buffer) was added to each well of the test plate. The test plate was incubated at 25 °C for 1 h. The final concentrations of the compounds were from 2 μM to 0.0256 nM, and the DMSO concentration was 1%.

e) After the incubation was completed, the test plate was washed twice with 200 μL of washing solution per well and then washed twice with 200 μL of PBS per well.

f) The Strep-HRP in the kit was 500-fold diluted with the 1× Strep-Diluent solution. The resulting solution was added to the test plate at 50 μL per well, and the test plate was incubated at 25 °C for 1 h.

g) After the incubation was completed, the test plate was washed twice with 200 μL of washing solution per well and then washed twice with 200 μL of PBS per well.

PeroxyGlow A and B in the kit were mixed at a ratio of 1:1, and the mixed solution was added to the test plate at 100 μL per well. Chemiluminescence was immediately read using a PerkinElmer Envision Multimode Plate Reader with an integration time of 0.5 s.

**Data analysis:** the original data was converted to inhibition rate using the equation (sample - Min)/(Max - Min) × 100%, and the $IC_{50}$ value was then fitted to a curve using four-parameter method (obtained from the "log(inhibitor) vs. response-variable slope" model in GraphPad Prism). Table 4 illustrates the enzymatic inhibitory activity of compound 1 disclosed herein against PARP1.

**Results:**

The PARP-1 kinase inhibitory activity of compound 1 disclosed herein were determined by the above method, and the obtained *in vitro* enzymatic inhibitory activity ($IC_{50}$) of compound 1 is shown in Table 4.

**Table 4. PARP-1 enzymatic activity of compound 1**

| Test compound | PARP1 ($IC_{50}$, nM) |
|---|---|
| Compound 1 | 2.3 |

**Conclusion:** Compound 1 exhibits excellent inhibitory activity against PARP1.

**Experimental Example 3. Anti-proliferation study on MDA-MB-436 CTG cells**

**Materials:** test compounds; RPMI-1640 medium; fetal bovine serum; penicillin/streptomycin antibiotic; MDA-MB-436 cells; Envision Multimode Plate Reader (PerkinElmer).

**Methodology:**

1. Procedures:

MDA-MB-436 cells were seeded in a white 96-well plate by adding 80 μL of cell suspension (containing 3000 MDA-MB-436 cells) to each well. The cell plate was incubated in a $CO_2$ incubator overnight.
The test compounds were serially 5-fold diluted to the $8^{th}$ concentration with a multichannel pipette, i.e., from 2 mM to 26 nM, in duplicate. 78 μL of medium was added to an intermediate plate, and the serially diluted compound was transferred to the intermediate plate at 2 μL per well. After being mixed well, the mixture was transferred to the cell plate at 20 μL per well. The cell plate was incubated in a $CO_2$ incubator for 7 days. Another cell plate was provided for reading signal values on the day of compound addition and these signal values were used as Max values in data analysis. Promega CellTiter-Glo was added to the cell plate at 25 μL per well and the luminescence signals were stabilized by incubation for 10 min at room temperature. Readings were taken using a PerkinElmer Envision Multimode Plate Reader.
Promega CellTiter-Glo was added to the cell plate at 25 μL per well and the luminescence signals were stabilized by incubation for 10 min at room temperature. Readings were taken using a PerkinElmer Envision Multimode Plate Reader.
2. Data analysis: the original data was converted to inhibition rate using the equation (sample - Min)/(Max - Min) × 100%, and the $IC_{50}$ value was then fitted to a curve using four-parameter method (obtained from the "log(inhibitor) vs. response-variable slope" model in GraphPad Prism). Table 5 illustrates the inhibitory activity of the compounds disclosed herein against MDA-MB-436 cell proliferation.
**Results:** The half maximal inhibitory concentrations ($IC_{50}$) of compound 1 and crystalline form A of the compound of formula (I) against proliferation *in vitro* are shown in Table 5.

**Table 5. Inhibitory activity of compound 1 and crystalline form A of compound of formula (I) against MDA-MB-436 cell proliferation**

| Compound | MDA-MB-436 ($IC_{50}$, nM) | Compound | MDA-MB-436 ($IC_{50}$, nM) |
|---|---|---|---|
| Compound 1 | 12.3 | Crystalline form A of compound of formula (I) | 8.4 |

**Conclusion:** Compound 1 and crystalline form A of the compound of formula (I) have excellent anti-proliferative activity against MDA-MB-436 cells with BRCA1 mutation, with crystalline form A of the compound of formula (I) showing significant anti-proliferative activity against MDA-MB-436 cells with BRCA1 mutation.

**Experimental Example 4. Anti-proliferation study on MDA-MB-231 CTG cells**

**Materials:** test compounds; R DMEM medium; fetal bovine serum; penicillin/streptomycin antibiotic; MDA-MB-231 cells; Envision Multimode Plate Reader.

**Procedures:**

MDA-MB-231 cells were seeded in a white 96-well plate by adding 80 μL of cell suspension (containing 5000 MDA-MB-231 cells) to each well. The cell plate was incubated in a $CO_2$ incubator overnight.
Eight concentrations were set for each compound. The test compounds were serially 3-fold diluted to the $8^{th}$ concentration with a multichannel pipette, i.e., from 2 mM to 920 nM, in duplicate. 78 μL of medium was added to an intermediate plate, and the serially diluted compound was transferred to the intermediate plate at 2 μL per well. After being mixed well, the mixture was transferred to the cell plate at 20 μL per well. The cell plate was incubated in a $CO_2$ incubator for 3 days. Another cell plate was provided for reading signal values on the day of compound addition and these signal values were used as Max values in data analysis. Promega CellTiter-Glo was added to the cell plate at 25 μL per well and the luminescence signals were stabilized by incubation for 10 min at room temperature. Readings were taken using a PerkinElmer Envision Multimode Plate Reader.

Promega CellTiter-Glo was added to the cell plate at 25 μL per well and the luminescence signals were stabilized by incubation for 10 min at room temperature. Readings were taken using a PerkinElmer Envision Multimode Plate Reader.

**Data analysis:** the original data was converted to inhibition rate using the equation **(sample - Min)/(Max - Min) × 100%**, and the $IC_{50}$ value was then fitted to a curve using four-parameter method (obtained from the "log(inhibitor) vs. response-variable slope" model in GraphPad Prism). Table 6 illustrates the inhibitory activity of the compound disclosed herein against MDA-MB-231 cell proliferation.

**Results:** The anti-proliferative activity of compound 1 against MDA-MB-231 cells with wild-type BRCA was determined by the above method, and the half maximal inhibitory concentration ($IC_{50}$) of compound 1 against proliferation *in vitro* is shown in Table 6.

**Table 6. Anti-proliferative activity of compound 1 against wild-type BRCA**

| Compound | MDA-MB-231 ($IC_{50}$, μM) |
|---|---|
| Compound 1 | > 10 |

**Conclusion:** Compound 1 has little inhibitory activity against MDA-MB-231 cells with wild-type BRCA, suggesting that compound 1 has excellent selectivity.

**Experimental Example 5. Anti-proliferation study on PARylation**

**Experimental materials:** test compounds, F12K medium, Lovo cells, Anti-Poly (ADP-ribose) mouse monoclonal antibody; FITC-labeled goat anti-mouse IgG; hydrogen peroxide; DAPI; PBS; methanol; acetone; Tween-20; skimmed milk powder; Envision Multimode Plate Reader.

**Preparation of reagents:**

Day One: Lovo cells were seeded on a plate at 60,000 cells/well, and then incubated overnight at 37 °C/5% COz. Day Two: The reagents were prepared:

1. Washing solution: Tween-20 was added to 1× PBS, and the final concentration of Tween-20 was 0.05%.

2. Blocking solution: Skimmed milk powder was added to the washing solution, and the final concentration of the skimmed milk powder was 5%.

3. Cell immobilization solution: Methanol and acetone were mixed in a ratio of 7:3.

Preparation of test compounds: Compound intermediate plate 1: The compound was diluted to final concentrations of 10 μM to 0.13 nM with DMSO and PBS, and the concentration of DMSO was 1%; Compound intermediate plate 2: The compound was diluted to final concentrations of 10 μM to 0.13 nM with DMSO and PBS containing 50 mM hydrogen peroxide, and the concentration of DMSO was 1%.

**Procedures:**

1. Cell supernatant was removed, and the compound was transferred from the compound intermediate plate 1 to the cell plate at 40 μL per well, and the cell plate was incubated at 37 °C for 30 min.

   Compound wells: compound, DMSO 1%;

   Negative and positive controls: 1% DMSO added;

   Blank controls: cell-free wells, PBS added.

2. After the incubation was completed, the compound was transferred from the compound intermediate plate 2 to the cell plate at 40 μL per well, and the final concentration of HzOz was 25 mM.

Compound wells: compound + 25 mM HzOz

Positive and negative controls: 1% DMSO + 25 mM HzOz

Blank controls: cell-free wells, PBS added

4. After the incubation was completed, the cell plate was washed once with PBS pre-cooled on ice and 100 μL of pre-cooled cell immobilization solution was added per well. After the cell plate was let stand at -20 °C for 10 min, the cell immobilization solution was discarded.

5. After air blast drying, the cell plate was washed with PBS at 200 μL per well, and then the PBS was discarded.

6. Blocking solution was added to the cell plate at 100 μL per well and the cell plate was incubated at 25 °C for 30 min, after which the blocking solution was discarded.

7. Anti-PAR antibody diluted in blocking solution at a ratio of 1:50 was added to the cell plate at 25 μL per well, and then the cell plate was incubated at 25 °C for 60 min.

Negative control wells: blocking solution added at 25 μL/well

Blank control wells: blocking solution added at 25 μL/well

8. After the incubation was completed, the cell plate was washed 4 times with washing solution at 200 μL per well, 3 min each time. The washing solution was then discarded.

9. Blocking solution containing 1:50 diluted FITC-conjugated goat anti-mouse IgG and 0.5 μg/mL DAPI was added to the cell plate at 25 μL per well, and then the cell plate was incubated at 25 °C for 60 min.

10. After the incubation was completed, the cell plate was washed 4 times with washing solution at 200 μL per well, 3 min each time.

11. After removal of the liquid, corresponding fluorescence values were read on Envision: FITC: 480 nm and 530 nm; DAPI: 360 nm and 460 nm.

**Data analysis:** The original data was normalized using the equation (FITC - negative control)/(DAPI - blank control), and the normalized data was converted to inhibition rate using the equation (sample - positive control)/(negative control - positive control) × 100%. The $IC_{50}$ value was then fitted to a curve using four-parameter method (obtained from the "log(inhibitor) vs. response-variable slope" model in GraphPad Prism). Table 7 illustrates the inhibitory activity of the compound disclosed herein.

**Result:** The half maximal inhibitory concentration ($IC_{50}$) of compound 1 against PARrylation is shown in Table 7.

**Table 7. Inhibitory activity of compound 1 against PARrylation**

| Test compound | Parylation ($IC_{50}$, nM) |
|---|---|
| Compound 1 | 19 |

**Conclusion:** Compound 1 has significant inhibitory activity against PARrylation.

**Experimental Example 6. Study on protein binding rate in plasma**

The protein binding rates of the compound disclosed herein in plasma of human, CD-1 mice and SD rats were determined. 796 μL of blank plasma was taken from human, CD-1 mice or SD rats, and 4 μL of test compound working solution (400 μM) or warfarin working solution (400 μM) was added to achieve a final concentration of 2 μM of both the test compound and the warfarin in the plasma sample. The samples were mixed well. The final concentration of organic phase DMSO was 0.5%; 50 μL of test compound and warfarin plasma sample was pipetted into a sample receiving plate (in triplicate), and a corresponding volume of blank plasma or buffer was immediately added such that the final volume in each sample well was 100 μL. The volume ratio of plasma to dialysis buffer was 1:1. 400 μL of stop solution

was added to these samples, which were used as T0 samples for determination of recovery rate and stability. The T0 samples were stored at 2-8 °C for subsequent treatment with other dialyzed samples; 150 μL of the test compound and warfarin plasma sample was added to an administration end of each dialysis well, and 150 μL of blank dialysis buffer was added to a corresponding receiving end of the dialysis well. The dialysis plate was then sealed with a gas permeable membrane, placed in a humid, 5% COz incubator and incubated at 37 °C for 4 h while shaking at about 100 rpm. After the dialysis was completed, 50 μL of the dialyzed buffer sample and dialyzed plasma sample were pipetted to a new sample receiving plate. A corresponding volume of blank plasma or buffer was added to the samples such that the final volume of each sample well was 100 μL, and the volume ratio of plasma to dialysis buffer was 1:1. All samples were subjected to LC/MS analysis after protein precipitation, and the protein binding rates and the recovery rates were calculated by the following formulas: protein unbinding rate (%) = 100 × drug concentration passing through dialysis membrane/drug concentration not passing through dialysate; protein binding rate (%) = 100 - protein unbinding rate (%); recovery rate (%) = 100 × (drug concentration passing through dialysis membrane + drug concentration not passing through dialysate)/total drug concentration before dialysis.

**Results:** The results are shown in Table 8.

**Table 8. Protein binding rates of compound 1 in plasma of different species**

| Test compound | Protein binding rate in plasma | | |
|---|---|---|---|
| | Human | CD-1 mouse | SD rat |
| Compound 1 | 85.1% | 89.6% | 92.3% |

**Conclusion:** Compound 1 has appropriate plasma protein binding rates.

**Experimental Example 7. Study on inhibition against cytochrome P450 isoenzymes**

The inhibition of the test compound against different subtypes of the human cytochrome P450 isoenzyme was determined. The test compound, a standard inhibitor (100× final concentration) and a mixed substrate working solution were prepared; the microsomes frozen in a refrigerator at -80 °C were taken out and thawed. 2 μL of a solution of the test compound and the standard inhibitor was added to corresponding wells, and 2 μL of a corresponding solvent was added to a non-inhibitor control (NIC) well and a blank control (Blank) well; then, 20 μL of a solution of mixed substrate was added to corresponding wells except for the Blank well (adding 20 μL of PB to the Blank well); a human liver microsome solution (labeled with the date after use and immediately putting back to a refrigerator) was prepared and then added to all wells at 158 μL per well; the sample plate was put into a 37 °C water bath for pre-incubation, and then a coenzyme factor (NADPH) solution was prepared; after 10 min, the NADPH solution was added to all the wells at 20 μL per well, and the sample plate was shaken to mix well and then incubated in a 37 °C water bath for 10 min; at corresponding time points, 400 μL of cold acetonitrile solution (internal standard: 200 ng/mL tolbutamide and labetalol) was added to stop the reaction; after being mixed well, the mixture in the sample plate was centrifuged at 4,000 rpm for 20 min, and proteins were precipitated; 200 μL of supernatant was collected and added into 100 μL of water, and the mixture was mixed well and then analyzed by LC/MS/MS.

**Results:** The results are shown in Table 9.

**Conclusion:** Compound 1 shows no or weak inhibitory effect against the 5 CYP enzymes.

**Table 9. Results of inhibition of compound 1 against cytochrome P450 isoenzymes**

| Test compound | $IC_{50}$(μM) | | | | |
|---|---|---|---|---|---|
| | CYP1A2 | CYP2C9 | CYP2C19 | CYP2D6 | CYP3A4-M |
| Compound 1 | >50 | >50 | 8.33 | 11.6 | 32.9 |

**Experimental Example 8. Metabolic stability in microsomes**

**Objective:** To test the metabolic stability of the test compound in liver microsomes of three species.

**Procedures:** 1 μM test compound and microsomes (0.5 mg/mL) were incubated at 37 °C in the presence of an NADPH regeneration system. Positive controls were testosterone (3A4 substrate), propylamine propiophenone (2D6 substrate) and diclofenac (2C9 substrate). At 37 °C, positive controls were incubated with microsomes (0.5 mg/mL) in the presence of an NADPH regeneration system. The reaction was stopped by direct mixing of the sample with cold acetonitrile containing an internal standard at various time points (0, 5, 10, 20, 30 and 60 min). The compound and

microsomes were incubated for 60 min in the absence of an NADPH regeneration system. One replicate (n = 1) was set at each time point. The samples were analyzed by LC/MS/MS; the concentration of the compound was characterized by the ratio of the analyte peak area to the internal standard peak area.

**Results:** The results are shown in Table 10.

**Table 10. Stability of compound 1 in liver microsomes of different species**

| Compound | Residual content after 60 min of incubation | | |
|---|---|---|---|
| | Human | Rat | Mouse |
| Compound 1 | 35.9% | 31.8% | 40.7% |

**Conclusion:** Compound 1 has moderate stability in all three species of rat, human and mouse.

**Experimental Example 9. Single-dose pharmacokinetic study in mice**

Objectives: To evaluate the pharmacokinetic behavior in male C57BL/6 mice and to determine the drug concentrations of the compound in the plasma, fiver and cerebrospinal fluid after a single dose.

Procedures: Healthy adult male C57BL/6 mice were selected for intragastric administration. The candidate compound was mixed with an appropriate amount of 10% DMSO/90% (20% hydroxypropyl-β-cyclodextrin), vortexed and sonicated to prepare a 0.5 mg/mL clear solution for later use. After the mice were administered intravenously at 1 mg/kg and orally at 5 mg/kg, whole blood was collected at certain time points. Plasma was separated, and liver and cerebrospinal fluid were collected. After pretreatment of the samples, the drug concentration was measured by LC-MS/MS, and pharmacokinetic parameters were calculated using Phoenix WinNonlin software. **Results:** The results are shown in Table 11.

**Conclusion:** Compound 1 has good $AUC_{0\text{-last}}$ and bioavailability in mice.

**Table 11. Results of pharmacokinetic study of compound 1 in mice and rats**

| Pharmacokinetic results (IV: 1 mg/kg PO: 5 mg/kg) | Rucaparib | Compound 1 |
|---|---|---|
| Clearance rate(mL/min/kg) | 99.9 | 41.0 |
| Apparent volume of distribution (L/kg) | 13.1 | 9.44 |
| $AUC_{0\text{-last}}$ (intravenous injection, nM. h) | 255 | 958 |
| $AUC_{0\text{-last}}$ (oral, nM. h) | 145 | 1186 |
| Half-life (h) | 1.78 | 3.02 |
| Maximum concentration (nM) | 24.8 | 240 |
| Bioavailability (%) | 14.6 | 24.8 |

**Experimental Example 10. *In vivo* pharmacodynamic study of test compound in BALB/c nude mouse model with subcutaneous human breast cancer MDA-MB-436 cell xenograft tumor**

**Objective:** To study the *in vivo* efficacy of the test compound in BALB/c nude mouse model with subcutaneous human breast cancer MDA-MB-436 cell xenograft tumor.

**Design:**

Table 12. Animal grouping and administration regimen of ***in vivo*** **pharmacodynamic study of compound 1**

| Groups | N1 | Compound | Dose (mg/kg) | Administration volume parameter (μL/g)[2] | Route of administration | Frequency of administration |
|---|---|---|---|---|---|---|
| 1 | 6 | Blank | -- | 10 | PO | QD × 28 days |
| 5 | 6 | Compound 1 | 12.5 | 10 | PO | QD × 28 days |

(continued)

| Groups | N1 | Compound | Dose (mg/kg) | Administration volume parameter (μL/g)[2] | Route of administration | Frequency of administration |
|---|---|---|---|---|---|---|
| 6 | 6 | Compound 1 | 25 | 10 | PO | QD × 28 days |
| 7 | 6 | Compound 1 | 50 | 10 | PO | QD × 28 days |
| Note: 1. N: the number of mice in each group; 2. administration volume: 10 μL/g based on the weight of mice. If the body weight decreased by more than 15%, the administration regimen should be adjusted accordingly. 3. QD: once daily; PO: oral administration. | | | | | | |

**Materials:** Age and body weight: female BALB/c nude mice, aged 6-8 weeks, 18-22 g. The study started after 3-7 days of adaptive feeding. The animal information card of each cage was labeled with the following information about the animals: number, sex, strain, receipt date, regime, study number, group and start of treatment. All the cages, padding and drinking water were sterilized before use. The cages, feed and drinking water were refreshed twice a week. The animals were identified with ear tags. Test samples: Nirapariband and compound 1. All the test samples were prepared with 10% DMSO + 90% (20% HP-β-CD) as vehicle, and the blank control group was administered with the vehicle alone.

**Procedures:**

1. Cell culturing: Human breast cancer MDA-MB-436 cells (ATCC, Manassas, VA, catalog No.: HTB-130) were cultured in an RPMI-1640 culture medium containing 10% fetal bovine serum and 1% Anti-anti through *in vitro* monolayer culture in an incubator at 37 °C/5% COz. The cells were digested with trypsin-EDTA twice a week for passaging as per conventional practice. At a cell saturation of 80%-90% and a required number, the cells were collected, counted and grafted.

2. Tumor cell grafting (tumor grafting): 0.2 mL ($1 \times 10^7$ cells) of MDA-MB-436 cells (along with matrigel in a volume ratio of 1:1) was subcutaneously grafting on the right back of each mouse, and the mice were randomly divided into groups for administration after the mean tumor volume was approximately 318 $mm^3$.

3. Daily observation of animals: the animals were monitored daily for health and death, and routine examinations include observation of the effect on tumor growth and the therapy on the daily performance of the animals, such as behavioral activities, food and water intake, change in body weight, appearance, or other abnormal conditions.

4. Tumor measurement and experimental indexes: the experimental indexes were to investigate whether the tumor growth was inhibited or delayed or the tumor was cured. Tumor diameters were measured twice weekly using a vernier caliper. The tumor volume was calculated using the following formula: $V = 0.5\ a \times b^2$, where a and b represent the long diameter and short diameter of the tumor, respectively. The anti-tumor efficacy of the compound was evaluated by TGI (%) or relative tumor proliferation rate T/C (%). TGI (%) refers to the tumor growth inhibition. Calculation of TGI (%): TGI (%) = [(1 - (mean tumor volume at the end of administration of a treatment group - mean tumor volume at the start of administration of the treatment group))/(mean tumor volume at the end of treatment of the solvent control group - mean tumor volume at the start of treatment of the solvent control group)] × 100%. The calculation formula for relative tumor proliferation rate T/C (%) was as follows: T/C (%) = $T_{RTV}/C_{RTV} \times 100\%$ ($T_{RTV}$: RTV of treatment group; $C_{RTV}$: RTV of negative control group). Relative tumor volume (RTV) was calculated based on the results of tumor measurement. The calculation formula was: RTV = Vt /V0, where V0 was the mean tumor volume measured at the time of grouping and administration (i.e., d0), Vt was the mean tumor volume at a certain measurement, and the data of $T_{RTV}$ and $C_{RTV}$ were obtained on the same day.

5. Statistical analysis: including mean and standard error of mean (SEM) of tumor volume at each time point in each group. The treatment group showed the best overall efficacy on day 27 after the administration at the end of study, and therefore statistical analysis was performed based on the data to evaluate the differences between the groups. The experimental data were analyzed using one-way ANOVA and Games-Howell method. All the data were analyzed using SPSS 17.0. $p < 0.05$ was defined as significance.

**Results:**

1. The body weights of the animals were used as a reference index for indirectly measuring the toxicity of the drug. In this model, no other morbidity or mortality occurred during the study.

2. Evaluation indexes of efficacy of test compound:

**Table 13. Evaluation of anti-tumor efficacy of test compound in subcutaneous human breast cancer MDA-MB-436 cell xenograft tumor model (calculated based on the tumor volume on day 27 after treatment)**

| Groups | Tumor volume ($mm^3$)[a] (day 27) | T/C[b] (%) | TGI[b] (%) | *p* value[c] *p* |
|---|---|---|---|---|
| Blank | 1586 ± 267 | -- | -- | -- |
| Compound 1 (12.5 mg/kg) | 377 ± 149 | 23.79 | 95.34 | 0.051 |
| Compound 1 (25 mg/kg) | 132 ± 32 | 8.31 | 114.69 | 0.025 |
| Compound 1 (50 mg/kg) | 91 ± 14 | 5.76 | 117.86 | 0.023 |

Note: a. Mean ± SEM. b. Tumor growth inhibition was calculated based on T/C and TGI. c. The p value is the significance of difference between the tumor volume in each treatment group and the tumor volume of the vehicle group on day 27.

**Conclusion:** The efficacy of compound 1 exhibits dose dependence at each given dosing gradient and excellent tumor growth inhibition. As can be seen from the change in body weight, the mice in the three dose groups of compound 1 have little change in body weight, indicating good tolerance.

**Experimental Example 11. Unit cell parameters of single crystal prepared from crystalline form A of the compound of formula (I)**

**Single crystal prepared from crystalline form A of the compound of formula (I):**

A 15-mg sample of crystalline form A of the compound of formula (I) was dissolved in 1 mL of ethanol/acetone/water (2:2:1) solution at room temperature, and the sample solution was placed in a 4 mL semi-sealed sample flask for slow evaporation at room temperature. On the third day, a yellow bulk crystal was obtained.

**Results:**

The ellipsoid plot of the three-dimensional structure of the single crystal prepared from crystalline form A of the compound of formula (I) is shown in FIG. 10, which is in R configuration.

**Example 12. Determination of chloride ion content in crystalline form A of the compound of formula (1) by potentiometric titration**

Materials: automatic potentiometric titrator, electronic balance, ultrapure water meter, silver nitrate titrant (0.1 mol/L)

Procedure:

1) 50 mL of purified water was added to a proper beaker and marked as Blank.
2) About 100 mg of crystalline form A of the compound of formula (I) was weighed precisely, added into a proper beaker and dissolved in 50 mL of purified water. The resulting solution is labeled as test sample solution.
3) The test sample solution and the blank solution were titrated with silver nitrate titrant (0.1 mol/L) according to the potentiometric titration method (General Chapter 0701, *Chinese Pharmacopoeia*, Volume IV). Every 1 mL of silver

nitrate titrant (0.1 mol/L) is equivalent to 3.545 mg of chlorine (Cl).

Calculation:

$$\text{Chloride ion content (\%)} = \frac{F \times (V_{SPL} - V_0)}{W_{SPL} \times 1000} \times 100\%$$

in the calculation formula:

F: titer, every 1 mL of silver nitrate titrant (0.1 mol/L) is equivalent to 3.545 mg of chlorine (Cl);
$W_{SPL}$: weight (g) of the test sample;
$V_{SPL}$: volume (mL) of the silver nitrate titrant (0.1 mol/L) consumed by the test sample solution;
$V_0$: volume (mL) of the silver nitrate titrant (0.1 mol/L) consumed by the blank solution.
Results: The chloride ion content was found to be 8.7% by calculation using the above formula.
Conclusion: According to the chloride ion content, it can be concluded that crystalline form A of the compound of formula (I) contains one hydrochloride salt.

## Claims

1. A compound of formula (I) or a crystalline form thereof:

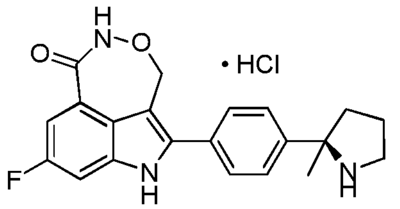

(I)

2. The compound of formula (I) or the crystalline form thereof according to claim 1, wherein the crystalline form comprises 2, 3 or more characteristic peaks selected from the group consisting of the diffraction peaks at the following 2Θ angles in an X-ray powder diffraction pattern using Cu Kα radiation: 8.59 ± 0.20°, 16.00 ± 0.20°, 20.76 ± 0.20°, 25.14 ± 0.20° and 25.96 ± 0.20°.

3. The compound of formula (I) or the crystalline form thereof according to claim 2, wherein the crystalline form comprises 12, 13, 14, 15 or more characteristic peaks selected from the group consisting of the diffraction peaks at the following 2Θ angles in an X-ray powder diffraction pattern using Cu Kα radiation: 8.59 ± 0.20°, 11.74 ± 0.20°, 12.34 ± 0.20°, 12.56 ± 0.20°, 13.32 ± 0.20°, 13.88 ± 0.20°, 14.70 ± 0.20°, 15.38 ± 0.20°, 16.00 ± 0.20°, 17.20 ± 0.20°, 18.80 ± 0.20°, 19.40 ± 0.20°, 19.72 ± 0.20°, 20.76 ± 0.20°, 21.30 ± 0.20°, 21.73 ± 0.20°, 22.24 ± 0.20°, 22.56 ± 0.20°, 23.50 ± 0.20°, 24.02 ± 0.20°, 25.14 ± 0.20°, 25.96 ± 0.20°, 26.84 ± 0.20°, 27.48 ± 0.20°, 28.20 ± 0.20°, 29.32 ± 0.20°, 30.36 ± 0.20°, 31.68 ± 0.20°, 31.98 ± 0.20°, 32.28 ± 0.20°, 32.90 ± 0.20°, 33.59 ± 0.20°, 34.72 ± 0.20°, 35.22 ± 0.20°, 35.98 ± 0.20°, 36.58 ± 0.20° and 38.48 ± 0.20°.

4. The compound of formula (I) or the crystalline form thereof according to claim 2 or 3, wherein the crystalline form has the following X-ray powder diffraction pattern data:

| No. | 2θ angle (± 0.2°) | Relative intensity (%) | No. | 2θ angle (± 0.2°) | Relative intensity (%) |
|---|---|---|---|---|---|
| 1 | 8.593 | 40.8 | 20 | 24.021 | 7.6 |
| 2 | 11.736 | 24.5 | 21 | 25.140 | 33.1 |
| 3 | 12.339 | 28.9 | 22 | 25.962 | 46.9 |

(continued)

| No. | 2θ angle (± 0.2°) | Relative intensity (%) | No. | 2θ angle (± 0.2°) | Relative intensity (%) |
|---|---|---|---|---|---|
| 4 | 12.559 | 38.8 | 23 | 26.836 | 4.2 |
| 5 | 13.315 | 6.3 | 24 | 27.478 | 10.7 |
| 6 | 13.875 | 5.4 | 25 | 28.197 | 9.7 |
| 7 | 14.701 | 2.4 | 26 | 29.323 | 5.7 |
| 8 | 15.376 | 7.5 | 27 | 30.362 | 3.0 |
| 9 | 15.999 | 41.4 | 28 | 31.679 | 10.6 |
| 10 | 17.202 | 16.5 | 29 | 31.981 | 6.0 |
| 11 | 18.795 | 3.0 | 30 | 32.281 | 5.8 |
| 12 | 19.397 | 18.1 | 31 | 32.900 | 2.9 |
| 13 | 19.720 | 4.1 | 32 | 33.586 | 2.2 |
| 14 | 20.759 | 100.0 | 33 | 34.723 | 4.4 |
| 15 | 21.299 | 4.5 | 34 | 35.220 | 2.9 |
| 16 | 21.726 | 4.8 | 35 | 35.977 | 3.5 |
| 17 | 22.241 | 9.8 | 36 | 36.578 | 1.3 |
| 18 | 22.562 | 7.7 | 37 | 38.479 | 2.7 |
| 19 | 23.498 | 4.8 | | | |

typically, the crystalline form has an X-ray powder diffraction pattern shown in FIG. 2.

5. The compound of formula (I) or the crystalline form thereof according to any one of claims 2-4, wherein the crystalline form has a starting point at 285.6 ± 5 °C and/or a peak value at 288.6 ± 5 °C of an exothermic peak in a differential scanning calorimetry curve; preferably, the crystalline form has a differential scanning calorimetry curve shown in FIG. 3.

6. The compound of formula (I) or the crystalline form thereof according to claim 1, wherein the crystalline form comprises 1 or more characteristic peaks selected from the group consisting of the diffraction peaks at the following 2Θ angles in an X-ray powder diffraction pattern using Cu Kα radiation: 15.86 ± 0.20°, 21.62 ± 0.20° and 23.54 ± 0.20°.

7. The compound of formula (I) or the crystalline form thereof according to claim 6, wherein the crystalline form comprises 7, 8, 9, 10 or more characteristic peaks selected from the group consisting of the diffraction peaks at the following 2Θ angles in an X-ray powder diffraction pattern using Cu Kα radiation: 11.52 ± 0.20°, 13.66 ± 0.20°, 14.28 ± 0.20°, 14.72 ± 0.20°, 15.86 ± 0.20°, 16.84 ± 0.20°, 18.30 ± 0.20°, 18.72 ± 0.20°, 21.62 ± 0.20°, 23.14 ± 0.20°, 23.54 ± 0.20°, 24.48 ± 0.20°, 24.84 ± 0.20°, 25.80 ± 0.20°, 26.28 ± 0.20°, 27.96 ± 0.20°, 28.40 ± 0.20°, 28.84 ± 0.20°, 29.82 ± 0.20°, 30.22 ± 0.20°, 31.74 ± 0.20°, 32.04 ± 0.20°, 32.69 ± 0.20°, 33.90 ± 0.20°, 34.50 ± 0.20°, 35.02 ± 0.20°, 36.08 ± 0.20°, 36.94 ± 0.20°, 37.46 ± 0.20° and 37.92 ± 0.20°.

8. The compound of formula (I) or the crystalline form thereof according to claim 6 or 7, wherein the crystalline form has the following X-ray powder diffraction pattern data:

| No. | 2θ angle (± 0.2°) | Relative intensity (%) | No. | 2θ angle (± 0.2°) | Relative intensity (%) |
|---|---|---|---|---|---|
| 1 | 11.519 | 5.5 | 16 | 27.962 | 18.8 |
| 2 | 13.659 | 10.9 | 17 | 28.402 | 5.4 |
| 3 | 14.276 | 7.4 | 18 | 28.844 | 3.3 |
| 4 | 14.721 | 22.4 | 19 | 29.819 | 3.8 |
| 5 | 15.860 | 63.9 | 20 | 30.220 | 5.7 |

(continued)

| No. | 2θ angle (± 0.2°) | Relative intensity (%) | No. | 2θ angle (± 0.2°) | Relative intensity (%) |
|---|---|---|---|---|---|
| 6 | 16.840 | 5.2 | 21 | 31.738 | 7.4 |
| 7 | 18.299 | 11.6 | 22 | 32.040 | 2.8 |
| 8 | 18.715 | 6.7 | 23 | 32.686 | 2.3 |
| 9 | 21.621 | 100.0 | 24 | 33.899 | 4.0 |
| 10 | 23.141 | 33.0 | 25 | 34.500 | 6.4 |
| 11 | 23.541 | 64.0 | 26 | 35.021 | 6.1 |
| 12 | 24.483 | 5.7 | 27 | 36.082 | 5.3 |
| 13 | 24.838 | 3.8 | 28 | 36.941 | 6.0 |
| 14 | 25.801 | 17.7 | 29 | 37.459 | 3.7 |
| 15 | 26.281 | 22.7 | 30 | 37.918 | 2.0 |

typically, the crystalline form has an X-ray powder diffraction pattern shown in FIG. 5.

**9.** The compound of formula (I) or the crystalline form thereof according to any one of claims 6-8, wherein the crystalline form has a starting point at 287.4 ± 5 °C and/or a peak value at 290.6 ± 5 °C of an exothermic peak in a differential scanning calorimetry curve; preferably, the crystalline form has a differential scanning calorimetry curve shown in FIG. 6.

**10.** The compound of formula (I) or the crystalline form thereof according to claim 1, wherein the crystalline form comprises 3, 4, 5, 6 or more characteristic peaks selected from the group consisting of the diffraction peaks at the following 2Θ angles in an X-ray powder diffraction pattern using Cu Kα radiation: 6.46 ± 0.20°, 18.78 ± 0.20°, 19.82 ± 0.20°, 20.98 ± 0.20°, 21.70 ± 0.20°, 23.30 ± 0.20° and 24.92 ± 0.20°.

**11.** The compound of formula (I) or the crystalline form thereof according to claim 10, wherein the crystalline form comprises 12, 13, 14, 15 or more characteristic peaks selected from the group consisting of the diffraction peaks at the following 2Θ angles in an X-ray powder diffraction pattern using Cu Kα radiation: 6.46 ± 0.20°, 9.59 ± 0.20°, 9.94 ± 0.20°, 10.58 ± 0.20°, 11.42 ± 0.20°, 12.44 ± 0.20°, 13.40 ± 0.20°, 14.86 ± 0.20°, 15.66 ± 0.20°, 16.32 ± 0.20°, 17.00 ± 0.20°, 17.54 ± 0.20°, 17.84 ± 0.20°, 18.78 ± 0.20°, 19.82 ± 0.20°, 20.22 ± 0.20°, 20.68 ± 0.20°, 20.98 ± 0.20°, 21.70 ± 0.20°, 22.32 ± 0.20°, 22.68 ± 0.20°, 23.30 ± 0.20°, 24.08 ± 0.20°, 24.92 ± 0.20°, 26.14 ± 0.20°, 26.86 ± 0.20°, 27.34 ± 0.20°, 27.82 ± 0.20°, 28.52 ± 0.20°, 29.34 ± 0.20°, 30.18 ± 0.20°, 31.36 ± 0.20° and 32.36 ± 0.20°.

**12.** The compound of formula (I) or the crystalline form thereof according to claim 10 or 11, wherein the crystalline form has the following X-ray powder diffraction pattern data:

| No. | 2θ angle (± 0.2°) | Relative intensity (%) | No. | 2θ angle (± 0.2°) | Relative intensity (%) |
|---|---|---|---|---|---|
| 1 | 6.460 | 100.0 | 18 | 20.979 | 52.1 |
| 2 | 9.593 | 13.4 | 19 | 21.702 | 50.5 |
| 3 | 9.941 | 20.9 | 20 | 22.321 | 40.0 |
| 4 | 10.582 | 17.7 | 21 | 22.683 | 45.0 |
| 5 | 11.422 | 17.3 | 22 | 23.300 | 51.0 |
| 6 | 12.438 | 34.1 | 23 | 24.080 | 37.4 |
| 7 | 13.402 | 6.3 | 24 | 24.921 | 71.2 |
| 8 | 14.859 | 18.3 | 25 | 26.139 | 21.0 |
| 9 | 15.664 | 15.9 | 26 | 26.855 | 27.8 |

(continued)

| No. | 2θ angle (± 0.2°) | Relative intensity (%) | No. | 2θ angle (± 0.2°) | Relative intensity (%) |
|---|---|---|---|---|---|
| 10 | 16.317 | 24.0 | 27 | 27.337 | 15.3 |
| 11 | 17.000 | 6.0 | 28 | 27.821 | 36.9 |
| 12 | 17.540 | 17.0 | 29 | 28.519 | 23.8 |
| 13 | 17.838 | 16.9 | 30 | 29.340 | 27.0 |
| 14 | 18.780 | 51.3 | 31 | 30.179 | 17.2 |
| 15 | 19.819 | 50.4 | 32 | 31.356 | 12.9 |
| 16 | 20.220 | 38.8 | 33 | 32.360 | 13.0 |
| 17 | 20.680 | 30.8 | | | |

typically, the crystalline form has an X-ray powder diffraction pattern shown in FIG. 8.

**13.** The compound of formula (I) or the crystalline form thereof according to any one of claims 10-12, wherein the crystalline form has a starting point at 261.6 ± 5 °C and/or a peak value at 267.5 ± 5 °C of an exothermic peak in a differential scanning calorimetry curve; preferably, the crystalline form has a differential scanning calorimetry curve shown in FIG. 9.

**14.** A crystalline composition of a compound of formula (I), wherein the crystalline form of the compound of formula (I) according to any one of claims 1-13 accounts for 50% or more, preferably 75% or more, more preferably 90% or more, and most preferably 95% or more of the crystalline composition by weight.

**15.** A pharmaceutical composition comprising a therapeutically effective amount of the compound of formula (I) or the crystalline form thereof according to any one of claims 1-13 and/or the crystalline composition according to claim 14, wherein the pharmaceutical composition may comprise at least one pharmaceutically acceptable carrier or other excipient.

**16.** Use of the compound of formula (I) or the crystalline form thereof according to any one of claims 1-13, the crystalline composition according to claim 14 and the pharmaceutical composition according to claim 15 in preparing a PARP inhibitor.

**17.** Use of the compound of formula (I) or the crystalline form thereof according to any one of claims 1-13, the crystalline composition according to claim 14 and the pharmaceutical composition according to claim 15 in preparing a medicament for preventing or treating a PARP receptor-associated disorder, wherein, preferably, the PARP receptor associated-disorder is selected from the group consisting of a tumor or a cancer; more preferably, the PARP receptor-associated disorder is breast cancer.

**18.** A method for preparing the compound of formula (I) or the crystalline form thereof according to any one of claims 2-4, wherein the method comprises: heating and stirring an amorphous form of the compound of formula (I) in an organic solvent, cooling for crystallization and filtering to give the crystalline form; the amorphous form of the compound of formula (I) has an X-ray powder diffraction pattern using Cu Kα radiation shown in FIG. 1; in the method for preparing the crystalline form, the organic solvent is selected from the group consisting of acetone, tetrahydrofuran and ethyl acetate, and preferably acetone.

**19.** The method for preparing the crystalline form according to claim 18, wherein the volume-to-molar ratio of the organic solvent to the amorphous form of the compound of formula (I) is (2-10) mL:1 mmol, and preferably (4-6) mL:1 mmol.

**20.** The method for preparing the crystalline form according to claim 18, wherein the amorphous form of the compound of formula (I) is prepared by stirring compound 1 in the presence of an organic solvent and hydrochloric acid, crystallizing and filtering; the organic solvent is selected from ethyl acetate; compound 1 has the following structure:

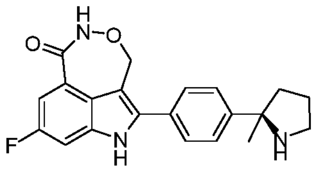

Compound 1 .

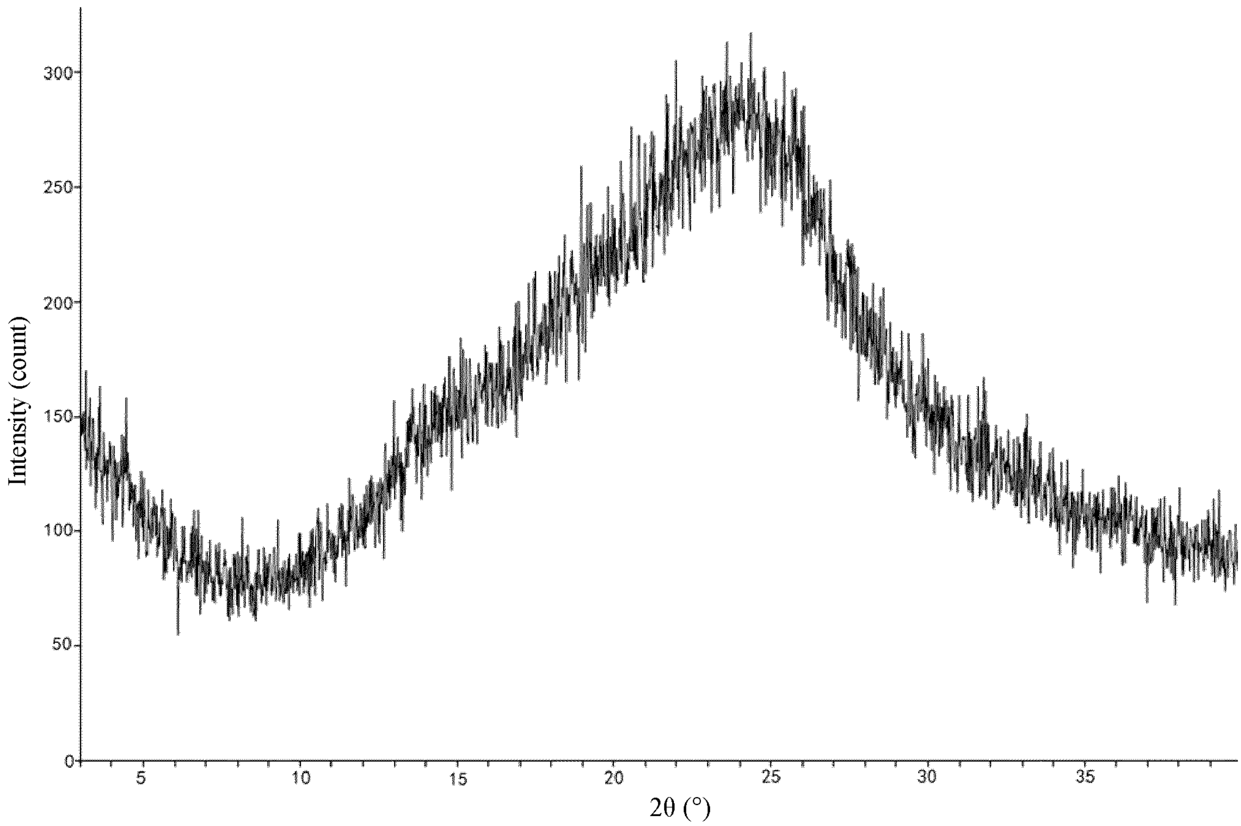
Intensity (count)
300
250
200
150
100
50
0
5
10
15
20
25
30
35
2θ (°)

FIG. 1

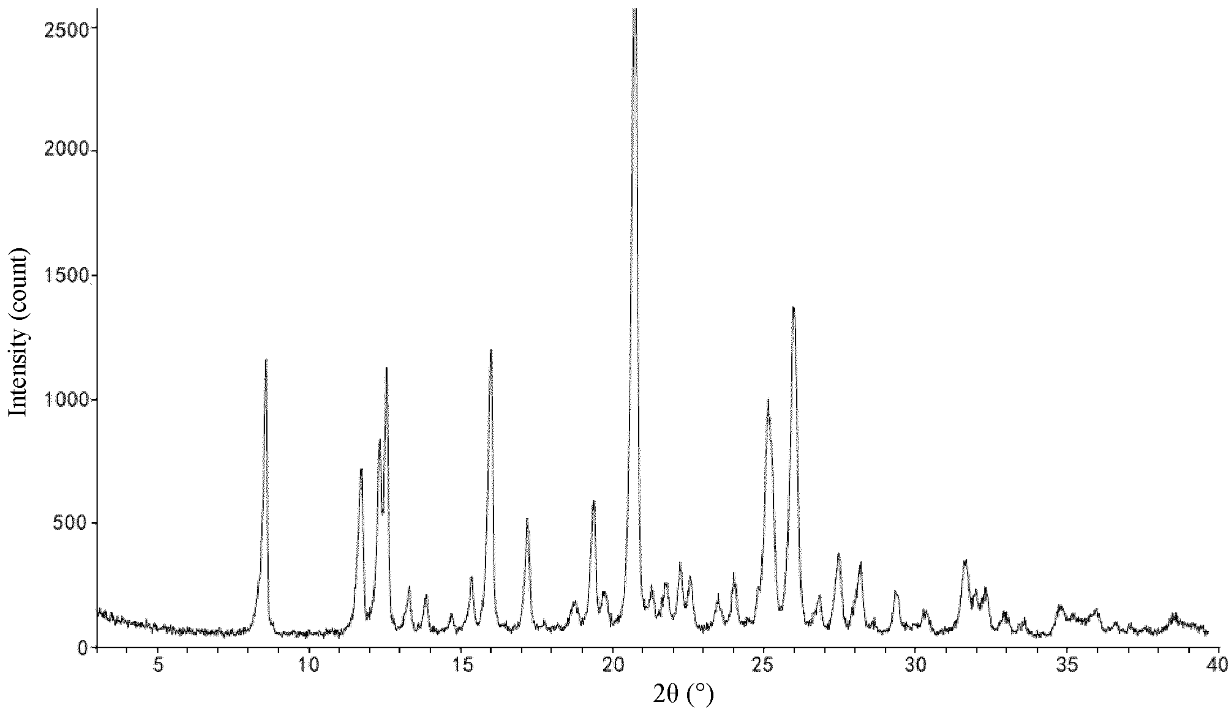
Intensity (count)
2500
2000
1500
1000
500
0
5
10
15
20
25
30
35
40
2θ (°)

FIG. 2

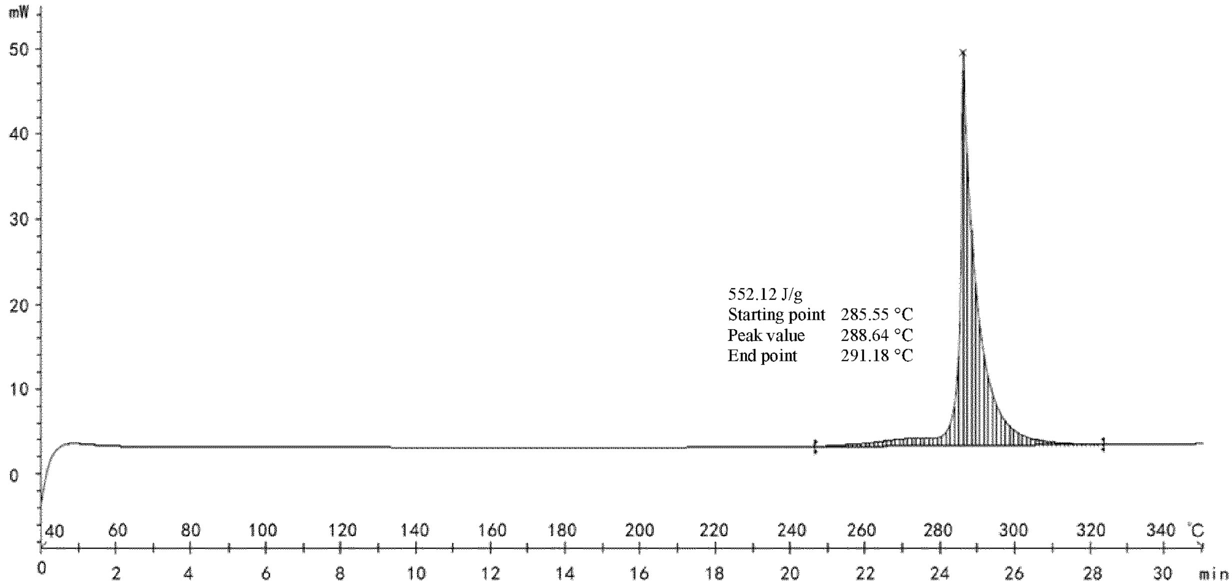
mW
50
40
30
20
10
0
552.12 J/g
Starting point 285.55 °C
Peak value 288.64 °C
End point 291.18 °C
40 60 80 100 120 140 160 180 200 220 240 260 280 300 320 340 °C
0 2 4 6 8 10 12 14 16 18 20 22 24 26 28 30 min

FIG. 3

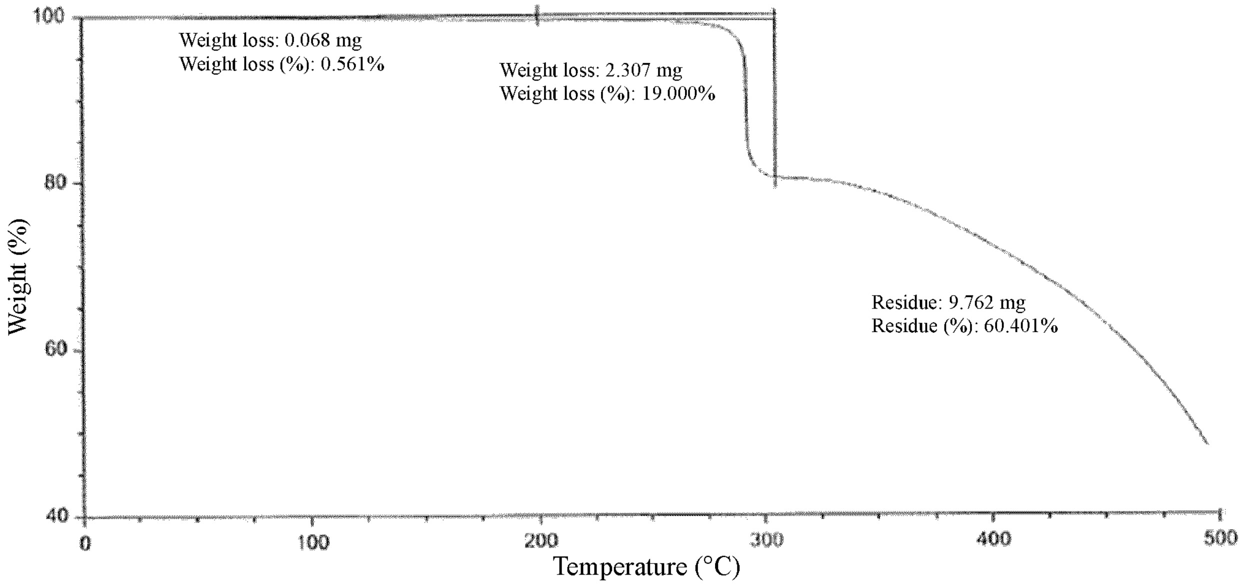
100
80
60
40
Weight (%)
Weight loss: 0.068 mg
Weight loss (%): 0.561%
Weight loss: 2.307 mg
Weight loss (%): 19.000%
Residue: 9.762 mg
Residue (%): 60.401%
0 100 200 300 400 500
Temperature (°C)

FIG. 4

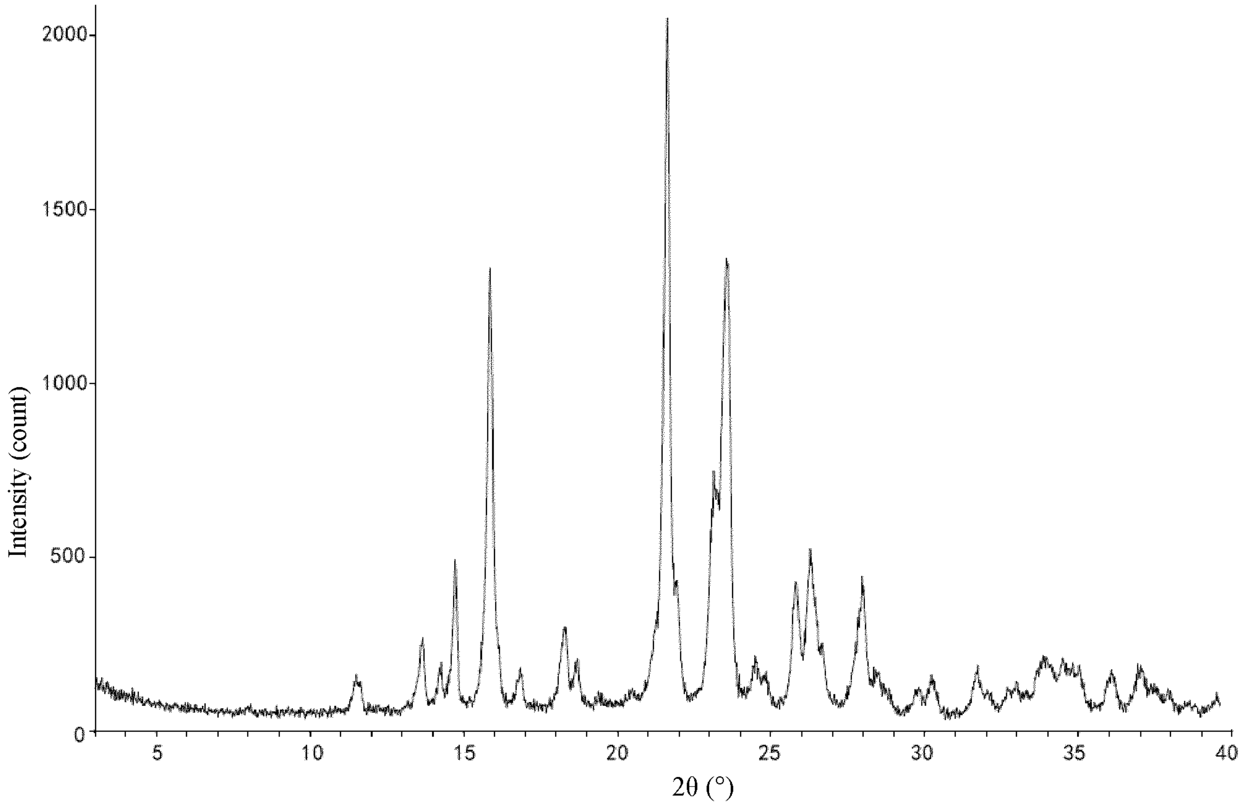
2000
1500
1000
500
0
Intensity (count)
5
10
15
20
25
30
35
40
2θ (°)

FIG. 5

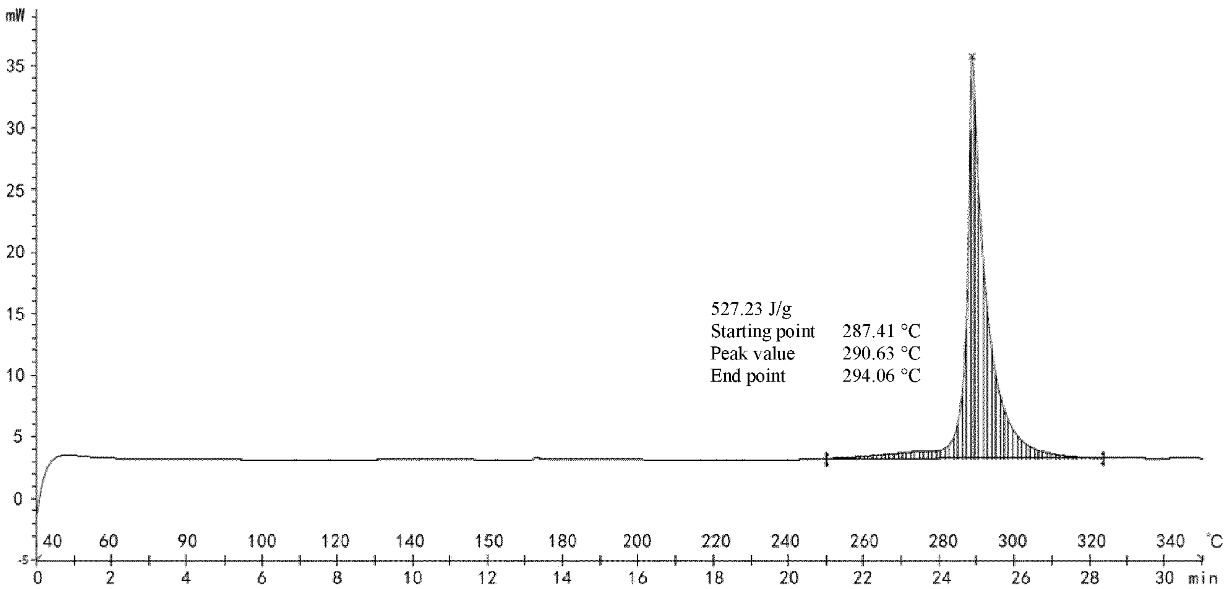
mW
35
30
25
20
15
10
5
0
-5
527.23 J/g
Starting point 287.41 °C
Peak value 290.63 °C
End point 294.06 °C
40 60 90 100 120 140 150 180 200 220 240 260 280 300 320 340 °C
0 2 4 6 8 10 12 14 16 18 20 22 24 26 28 30 min

FIG. 6

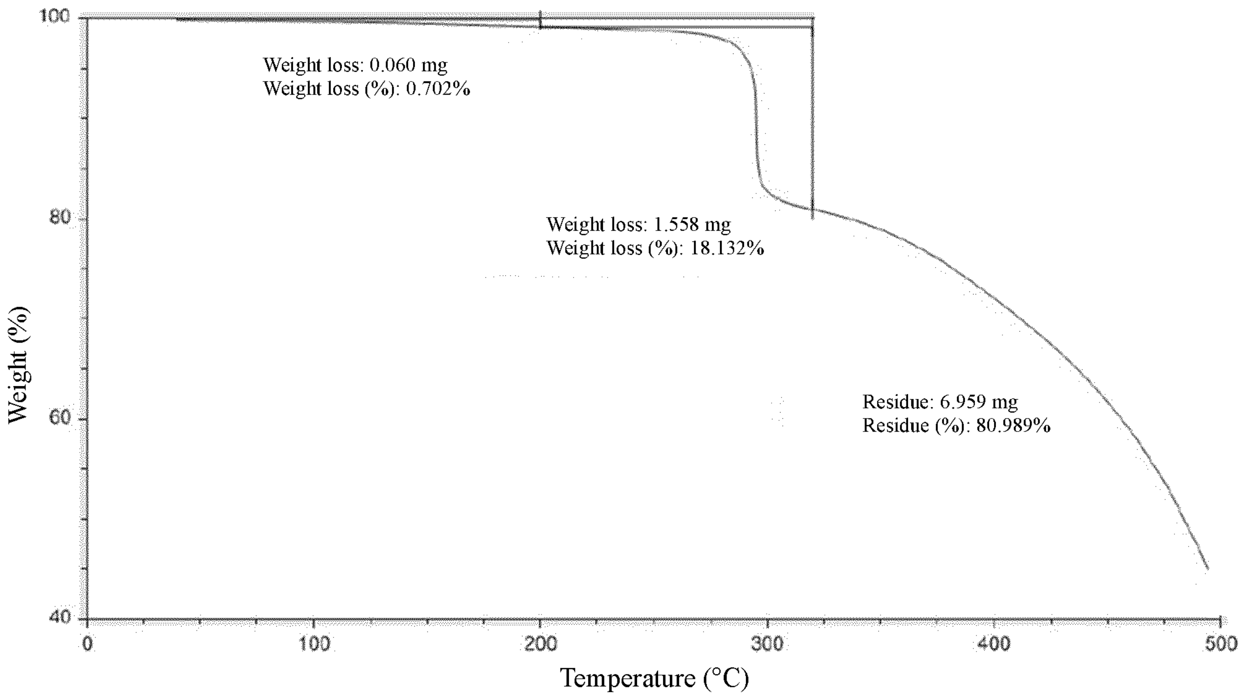
Weight loss: 0.060 mg
Weight loss (%): 0.702%
Weight loss: 1.558 mg
Weight loss (%): 18.132%
Residue: 6.959 mg
Residue (%): 80.989%
Weight (%)
100
80
60
40
0
100
200
300
400
500
Temperature (°C)

FIG. 7

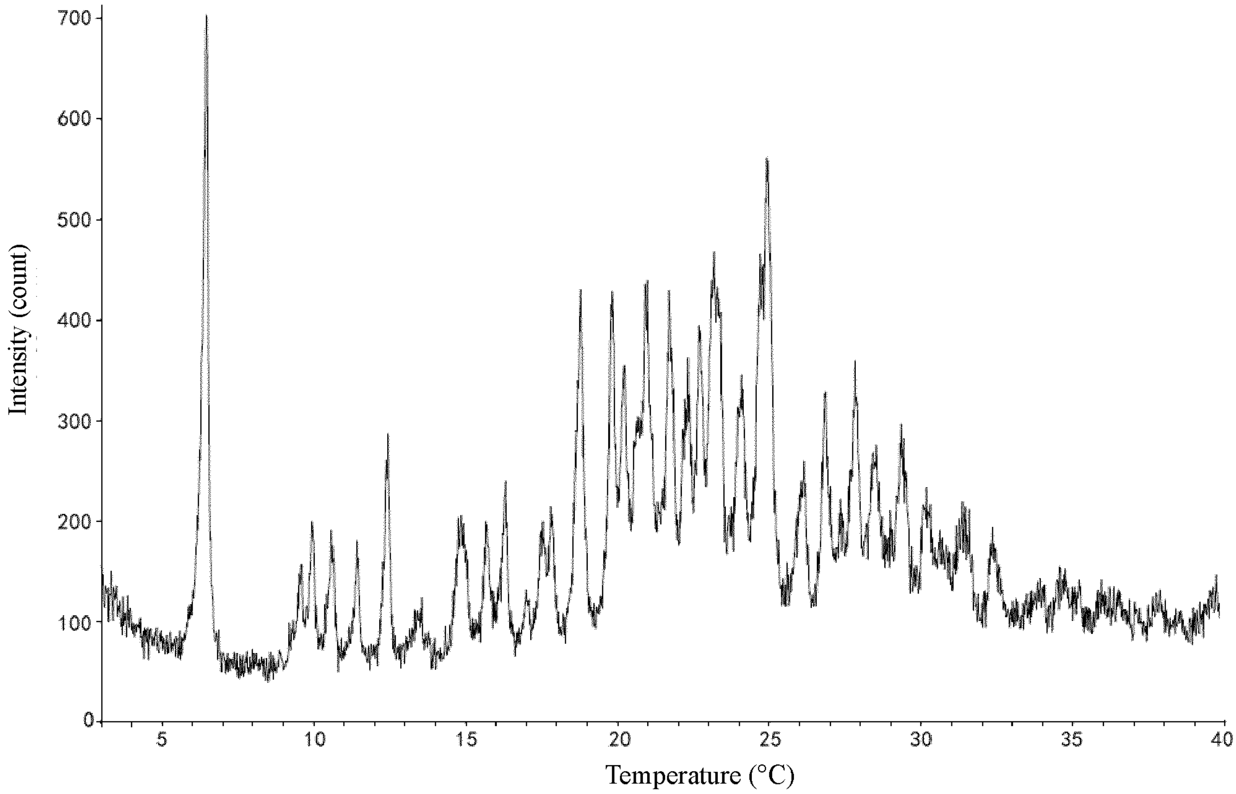
Intensity (count)
700
600
500
400
300
200
100
0
5
10
15
20
25
30
35
40
Temperature (°C)

FIG. 8

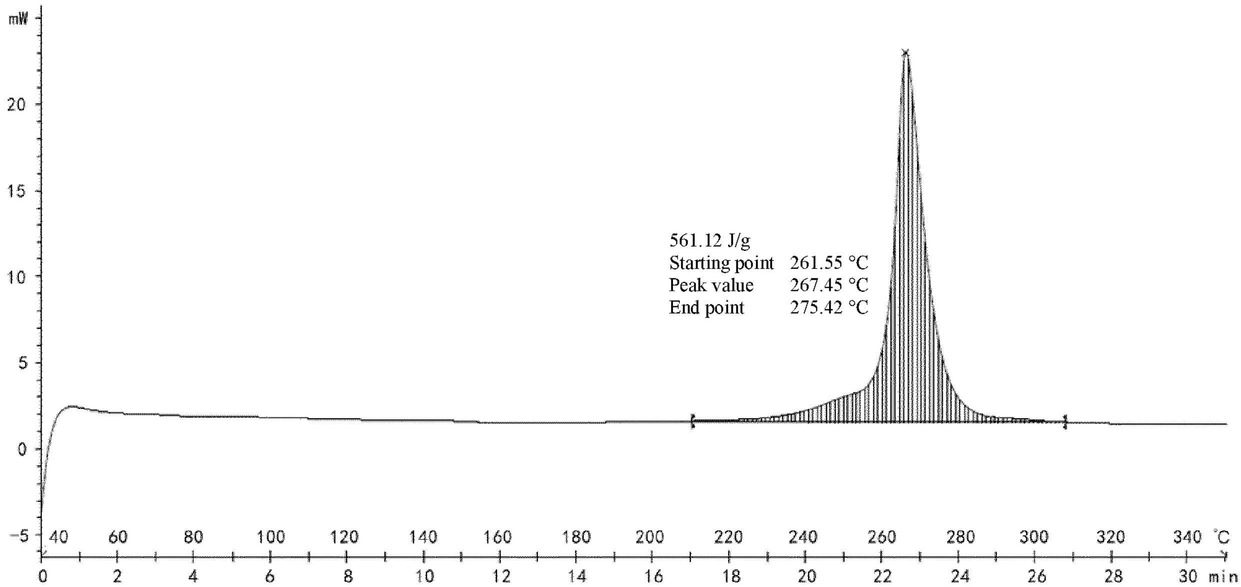
mW
20
15
10
5
0
−5
561.12 J/g
Starting point 261.55 °C
Peak value 267.45 °C
End point 275.42 °C
40 60 80 100 120 140 160 180 200 220 240 260 280 300 320 340 ℃
0 2 4 6 8 10 12 14 16 18 20 22 24 26 28 30 min

FIG. 9

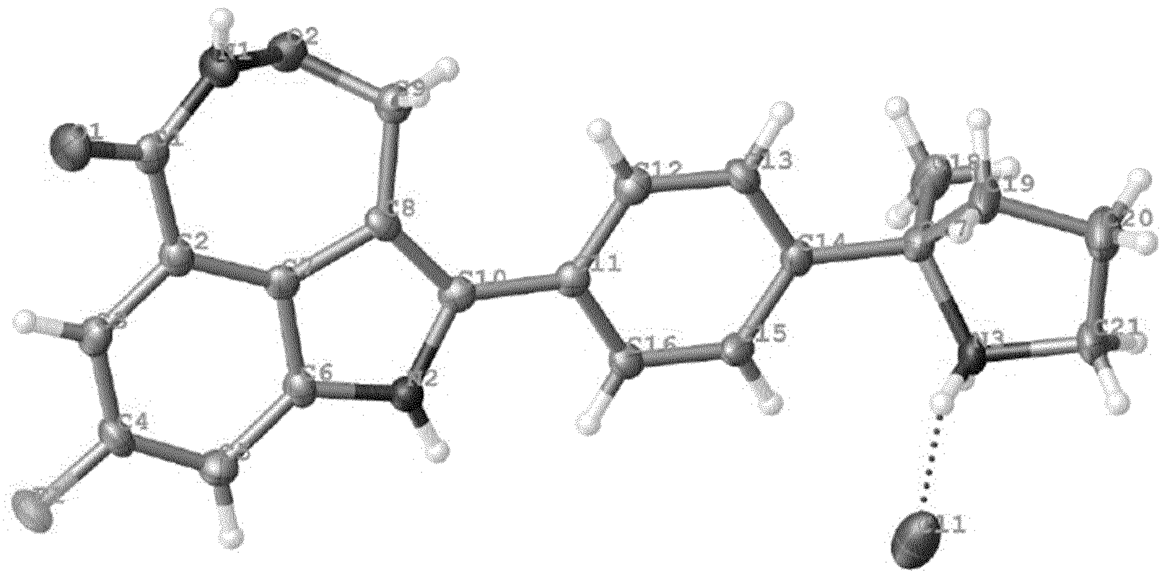

FIG. 10

**INTERNATIONAL SEARCH REPORT**

International application No.
**PCT/CN2021/109452**

**A. CLASSIFICATION OF SUBJECT MATTER**

C07D 498/06(2006.01)i; C07D 267/02(2006.01)i; A61K 31/55(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

EPODOC, WPI, CNPAT, CNKI, ISI web of science, STN, 正大天晴药业集团股份有限公司, 胡彦宾, 抑制剂, 吲哚, 酰肟, 并, 稠, 氮, 氧, 聚(ADP－核糖基)转化酶, 结晶, 晶体, 癌, 肿瘤, 制备, PARP, cancer, prepare, Indolo, heptamyl, oxime, inhibitor, indole, seven, member, acyloxime, pharmaceutical, composition, inflammatory, disease, structure search

**C. DOCUMENTS CONSIDERED TO BE RELEVANT**

| Category* | Citation of document, with indication, where appropriate, of the relevant passages | Relevant to claim No. |
|---|---|---|
| A | CN 1342161 A (AGOURON PHARMACEUTICALS INC. et al.) 27 March 2002 (2002-03-27)<br>description 3-4, 12-22 and pages 30-94 | 1-20 |
| A | CN 1374961 A (BASF AG) 16 October 2002 (2002-10-16)<br>description pages 2 and 3 | 1-20 |
| A | CN 102869258 A (BIOMARIN PHARMACEUTICIAL INC.) 09 January 2013 (2013-01-09)<br>description, pages 1-2, and invention name | 1-20 |
| A | CN 1759118 A (PRIZER INC.) 12 April 2006 (2006-04-12)<br>description, pages 2-6 | 1-20 |
| PX | WO 2020156577 A1 (CHIA TAI TIANQING PHARMACEUTICAL GROUP CO., LTD. et al.) 06 August 2020 (2020-08-06)<br>description, pages 1-47 | 1-20 |
| PX | WO 2021018298 A1 (MEDSHINE DISCOVERY INC.) 04 February 2021 (2021-02-04)<br>description, pages 1-16 | 1-20 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 September 2021** | **29 October 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/109452**

| Patent document cited in search report | Publication date (day/month/year) | Patent family member(s) | Publication date (day/month/year) |
|---|---|---|---|
| CN 1342161 A | 27 March 2002 | HK 1040992 A1 | 28 June 2002 |
| | | CZ 302941 B6 | 25 January 2012 |
| | | RS 50031 B | 28 November 2008 |
| | | TR 200102005 T2 | 21 December 2001 |
| | | LV 12770 A | 20 December 2001 |
| | | EE 05006 B1 | 15 April 2008 |
| | | EP 1140936 B1 | 17 March 2004 |
| | | US 7429578 B2 | 30 September 2008 |
| | | US 6977298 B2 | 20 December 2005 |
| | | OA 11749 A | 19 July 2005 |
| | | IS 5995 A | 10 July 2001 |
| | | YU 49001 A | 28 November 2005 |
| | | US 2005085460 A1 | 21 April 2005 |
| | | NO 20013313 L | 10 September 2001 |
| | | SK 9662001 A3 | 04 April 2002 |
| | | WO 0042040 A1 | 20 July 2000 |
| | | EA 200100764 A1 | 27 June 2002 |
| | | NO 20013313 D0 | 04 July 2001 |
| | | ES 2218110 T3 | 16 November 2004 |
| | | AP 1538 A | 10 January 2006 |
| | | ID 30138 A | 08 November 2001 |
| | | HU 229875 B1 | 28 November 2014 |
| | | HR P20010573 B1 | 30 April 2006 |
| | | CA 2360003 C | 10 July 2012 |
| | | NO 320343 B1 | 28 November 2005 |
| | | NO 20013313 A | 10 September 2001 |
| | | BR 0008614 B1 | 25 November 2014 |
| | | DK 1140936 T3 | 21 June 2004 |
| | | IL 144112 D0 | 23 May 2002 |
| | | US 2006009517 A1 | 12 January 2006 |
| | | AT 261963 T | 15 April 2004 |
| | | JP 4093448 B2 | 04 June 2008 |
| | | DE 60009033 T2 | 05 August 2004 |
| | | PL 357049 A1 | 12 July 2004 |
| | | PT 1140936 E | 30 June 2004 |
| | | EA 004989 B1 | 28 October 2004 |
| | | EE 200100364 A | 15 October 2002 |
| | | US 6495541 B1 | 17 December 2002 |
| | | PL 210415 B1 | 31 January 2012 |
| | | NZ 512731 A | 30 January 2004 |
| | | BG 105811 A | 31 May 2002 |
| | | HU 0105414 A3 | 28 April 2003 |
| | | CN 100418967 C | 17 September 2008 |
| | | OA 12185 A | 09 May 2006 |
| | | CA 2360003 A1 | 20 July 2000 |
| | | HU 0105414 A2 | 29 May 2002 |
| | | EP 1140936 A1 | 10 October 2001 |
| | | IL 144112 A | 05 July 2006 |
| | | AP 200102211 A0 | 30 September 2001 |
| CN 1374961 A | 16 October 2002 | CA 2352194 A1 | 05 April 2001 |

Form PCT/ISA/210 (patent family annex) (January 2015)

**INTERNATIONAL SEARCH REPORT**
**Information on patent family members**

International application No.
**PCT/CN2021/109452**

| Patent document cited in search report | Publication date (day/month/year) | Patent family member(s) | Publication date (day/month/year) |
|---|---|---|---|
| | | NO 20012567 L | 25 June 2001 |
| | | JP 2003510328 A | 18 March 2003 |
| | | SK 8842001 A3 | 07 January 2002 |
| | | BG 105650 A | 28 February 2002 |
| | | NO 20012567 A | 25 June 2001 |
| | | BR 0007174 A | 04 September 2001 |
| | | HK 1048999 A1 | 25 April 2003 |
| | | CZ 20012373 A3 | 15 May 2002 |
| | | NO 20012567 D0 | 25 May 2001 |
| | | IL 143349 D0 | 21 April 2002 |
| | | HU 0104917 A3 | 28 December 2002 |
| | | WO 0123390 A2 | 05 April 2001 |
| | | HU 0104917 A2 | 29 April 2002 |
| | | WO 0123390 A3 | 27 December 2001 |
| | | KR 20010087401 A | 15 September 2001 |
| | | AU 1271201 A | 30 April 2001 |
| | | TR 200101499 T1 | 23 September 2002 |
| | | PL 347885 A1 | 22 April 2002 |
| | | EP 1183259 A2 | 06 March 2002 |
| CN 102869258 A | 09 January 2013 | WO 2011097334 A1 | 11 August 2011 |
| | | EP 2531033 A4 | 31 July 2013 |
| | | US 8541403 B2 | 24 September 2013 |
| | | AU 2011212928 B2 | 23 June 2016 |
| | | JP 2020176136 A | 29 October 2020 |
| | | US 2015209363 A1 | 30 July 2015 |
| | | JP 2019011356 A | 24 January 2019 |
| | | US 2019070189 A1 | 07 March 2019 |
| | | AU 2016231573 A1 | 20 October 2016 |
| | | EP 2531033 A1 | 12 December 2012 |
| | | US 10493078 B2 | 03 December 2019 |
| | | JP 2013518892 A | 23 May 2013 |
| | | US 2014066429 A1 | 06 March 2014 |
| | | CA 2787844 A1 | 11 August 2011 |
| | | JP 5883397 B2 | 15 March 2016 |
| | | AU 2018206826 A1 | 09 August 2018 |
| | | US 9018201 B2 | 28 April 2015 |
| | | CN 106943406 A | 14 July 2017 |
| | | US 2011190288 A1 | 04 August 2011 |
| | | AU 2011212928 A1 | 09 August 2012 |
| | | JP 2017197565 A | 02 November 2017 |
| | | JP 2016128492 A | 14 July 2016 |
| | | CA 2787844 C | 27 August 2019 |
| CN 1759118 A | 12 April 2006 | HK 1086257 A1 | 15 September 2006 |
| | | BR PI0406701 A | 20 December 2005 |
| | | AP 2048 A | 24 September 2009 |
| | | IL 169082 A | 28 February 2011 |
| | | EP 1585749 A1 | 19 October 2005 |
| | | SI EP1585749 T1 | 31 October 2008 |
| | | IS 7884 A | 09 June 2005 |
| | | US 6967198 B2 | 22 November 2005 |

Form PCT/ISA/210 (patent family annex) (January 2015)

**INTERNATIONAL SEARCH REPORT**
**Information on patent family members**

International application No.
**PCT/CN2021/109452**

| Patent document cited in search report | Publication date (day/month/year) | Patent family member(s) | Publication date (day/month/year) |
|---|---|---|---|
| | | NO 20053775 D0 | 08 August 2005 |
| | | US 7462713 B2 | 09 December 2008 |
| | | JP 3990718 B2 | 17 October 2007 |
| | | ZA 200504674 B | 26 July 2006 |
| | | BR 0406701 A | 20 December 2005 |
| | | CA 2512683 C | 16 March 2010 |
| | | AP 200503353 D0 | 30 September 2005 |
| | | ZA 200504674 A | 26 July 2006 |
| | | OA 13017 A | 10 November 2006 |
| | | DK 1585749 T3 | 22 September 2008 |
| | | EA 200500893 A1 | 24 February 2006 |
| | | CA 2512683 A1 | 29 July 2004 |
| | | EP 1947102 A1 | 23 July 2008 |
| | | CR 7899 A | 05 August 2005 |
| | | DE 602004015724 D1 | 25 September 2008 |
| | | CN 1759118 B | 08 December 2010 |
| | | JP 2006516274 A | 29 June 2006 |
| | | TN SN05176 A1 | 11 June 2007 |
| | | AU 2004203977 B2 | 17 June 2010 |
| | | SI 1585749 T1 | 31 October 2008 |
| | | NO 20053775 L | 16 September 2005 |
| | | AP 200503353 A0 | 30 September 2005 |
| | | NZ 540638 A | 21 December 2007 |
| | | US 2005075499 A1 | 07 April 2005 |
| | | WO 2004063198 A1 | 29 July 2004 |
| | | CY 1108408 T1 | 12 February 2014 |
| | | EP 1585749 B1 | 13 August 2008 |
| | | EC SP055911 A | 22 November 2005 |
| | | NO 20053775 A | 16 September 2005 |
| | | US 2007135415 A1 | 14 June 2007 |
| | | RS 20050522 A | 31 December 2007 |
| | | AP 2005003353 A0 | 30 September 2005 |
| | | US 2006004052 A1 | 05 January 2006 |
| | | EA 009337 B1 | 28 December 2007 |
| | | US 7132533 B2 | 07 November 2006 |
| | | PT 1585749 E | 23 October 2008 |
| | | AU 2004203977 A1 | 29 July 2004 |
| | | UA 80733 C2 | 25 October 2007 |
| | | KR 100697746 B1 | 22 March 2007 |
| | | GE P20084367 B | 13 May 2008 |
| | | HR P20050624 A2 | 28 February 2006 |
| WO 2020156577 A1 | 06 August 2020 | CN 113365998 | 07 September 2021 |
| WO 2021018298 A1 | 04 February 2021 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202010762484
- CN 202010772764

### Non-patent literature cited in the description

- *Nature,* 2005, 913-917
- *J. Med. Chem.,* 2010, 4561